# EUROPEAN PATENT SPECIFICATION

(11) **EP 2 958 590 B1**
(45) Date of publication and mention of the grant of the patent: **07.04.2021**
(21) Application number: 14709738.0
(22) Date of filing: 24.02.2014
(51) Int. Cl.: A61K 39/395, A61P 35/00

(54) **BISPECIFIC ANTIBODY BINDING TO NAALADL2 AND CD3 FOR USE AS A THERAPEUTIC IN TREATING PROSTATE CANCER**
BISPEZIFISCHER ANTIKÖRPER, DER AN NAALADL2 UND CD3 BINDET, ZUR VERWENDUNG ALS THERAPEUTIKUM BEI DER BEHANDLUNG VON PROSTATAKREBS
ANTICORPS BISPÉCIFIQUE QUI SE LIE AU NAALADL2 ET AU CD3 POUR UNE UTILISATION THÉRAPEUTIQUE DANS LE TRAITEMENT DU CANCER DE LA PROSTATE

(30) Priority: 25.02.2013 GB 201303308
(43) Date of publication of application: 30.12.2015
(73) Proprietor: Oxford Bio Therapeutics Limited, Milton Park Abingdon Oxfordshire OX14 4RZ (GB)
(72) Inventor: HUDSON, Lindsey Jane, Abingdon Oxfordshire OX14 4RZ (GB); ACKROYD, James Edward, Abingdon Oxfordshire OX14 4RZ (GB)
(86) International application number: PCT/GB2014/050549
(87) International publication number: WO 2014/128504

(56) References cited:
- WO-A1-94/13804
- WO-A1-2009/028521
- WO-A1-2011/121110
- WO-A2-2005/040402
- KROESEN ET AL: "Approaches to lung cancer treatment using the CD3 x EGP-2-directed bispecific monoclonal antibody BIS-1.", CANCER IMMUNOLOGY, IMMUNOTHERAPY, vol. 45, no. 3-4, 1 January 1997 (1997-01-01), pages 203-206, XP055110107, ISSN: 0340-7004
- KERSTIN FORTMÜLLER ET AL: "Effective targeting of prostate cancer by lymphocytes redirected by a PSMA * CD3 bispecific single-chain diabody", PROSTATE, WILEY-LISS, NEW YORK, NY, US, vol. 71, no. 6, 1 May 2011 (2011-05-01), pages 588-596, XP002648688, ISSN: 0270-4137, DOI: 10.1002/PROS.21274 [retrieved on 2010-10-13]
- KOSUKE YAMAMOTO ET AL: "A novel bispecific single-chain antibody for ADAM17 and CD3 induces T-cell-mediated lysis of prostate cancer cells", BIOCHEMICAL JOURNAL, vol. 402, no. 1, 15 June 2012 (2012-06-15) , pages 884-144, XP055112455, ISSN: 0264-6021, DOI: 10.1016/j.ccr.2012.01.017
- H C WHITAKER ET AL: "N-acetyl-L-aspartyl-L-glutamate peptidase-like 2 is overexpressed in cancer and promotes a pro-migratory and pro-metastatic phenotype", ONCOGENE, 1 November 2013 (2013-11-01), XP55119546, ISSN: 0950-9232, DOI: 10.1038/onc.2013.464
- CHAMES PATRICK ET AL: "Bispecific antibodies for cancer therapy. The light at the end of the tunnel?", MABS, LANDES BIOSCIENCE, US, vol. 1, no. 6, 1 November 2009 (2009-11-01), pages 539-547, XP002688758, ISSN: 1942-0862

## Description

### INTRODUCTION

The present invention relates to the identification of a membrane protein associated with cancer, such as prostate cancer, bladder cancer, breast cancer, esophagus cancer, head and neck cancer, colorectal cancer, liver cancer, lung cancer, ovarian cancer, gastric cancer, uterus cancer and/or pancreatic cancer which has utility as a therapeutic target for the treatment of cancers or as a marker for cancers. In particular, the protein represents a biological target against which affinity reagents including therapeutic antibodies, or other pharmaceutical agents, can be made. The invention also relates to the use of such affinity reagents for the treatment and/or diagnosis of cancers.

### BACKGROUND OF THE INVENTION

The major challenges in treatment of cancer, such as prostate cancer, bladder cancer, breast cancer, esophagus cancer, head and neck cancer, colorectal cancer, liver cancer, lung cancer, ovarian cancer, gastric cancer, uterus cancer and pancreatic cancer are to improve early detection rates, to find new non-invasive markers that can be used to follow disease progression and identify relapse, and to find improved and less toxic therapies, especially for more advanced disease where 5 year survival is still poor. There is a great need to identify targets which are more specific to the cancer cells, e.g. ones which are expressed on the surface of the tumour cells so that they can be attacked by promising new approaches like immunotherapeutics and targeted toxins.

Inactive N-acetylated-alpha-linked acidic dipeptidase-like protein 2 is a member of the peptidase M28 family. Protein expression of Inactive N-acetylated-alpha-linked acidic dipeptidase-like protein 2 on cancer cells, which would be required to demonstrate its utility as a cell-surface target for e.g. antibody-based cancer therapies, for example, has not previously been disclosed.

### SUMMARY OF THE INVENTION

The present invention is defined in the claims.

The disclosure describes the detection of Inactive N-acetylated-alpha-linked acidic dipeptidase-like protein 2, hereinafter referred to as NAALADL2, in membrane extracts of various disease tissues, e.g. prostate cancer, bladder cancer, breast cancer, esophagus cancer, head and neck cancer, colorectal cancer, liver cancer, lung cancer, ovarian cancer, gastric cancer, uterus cancer and pancreatic cancer.

The differential expression of NAALADL2 in various cancers permits the protein to be targeted using affinity reagent-, e.g. antibody-, based therapies for such cancers. Thus NAALADL2 can be used in the generation of affinity reagents, including antibodies, that bind specifically to epitopes within NAALADL2, and can be targeted by such affinity reagents as the basis of treatment. Affinity reagents, including antibodies, that target a protein on the cell surface of cancer cells may be employed in the treatment of cancer through a variety of mechanisms, including (i) lysis by complement mediated or antibody-dependent cellular cytotoxicity (ADCC), (ii) lysis by drugs or toxin(s) conjugated to such affinity reagents or (iii) inhibition of the physiological function of such protein, which may be driving growth of cancer cells, e.g. through signaling pathways. An important aspect of such affinity reagent-based treatment is that the normal expression profile of the protein target, in terms of tissue distribution and expression level, is such that any targeting of the protein target on normal tissues by the antibody does not give rise to adverse side-effects through binding to normal tissues.

Described herein is a method for the treatment or prophylaxis of cancer wherein NAALADL2 is expressed in said cancer, which comprises administering to a subject in need thereof a therapeutically effective amount of an affinity reagent which binds to NAALADL2.

The invention provides a bispecific antibody which binds to NAALADL2 and CD3 for use in the treatment or prophylaxis of prostate cancer.

The description also describes the use of an affinity reagent which binds to NAALADL2 in the manufacture of a medicament for the treatment or prophylaxis of cancer, preferably wherein the cancer is one of the diseases of the invention.

The bispecific antibodies for use in the invention preferably bind specifically to NAALADL2.

The described affinity reagent may be an antibody, e.g. a whole antibody, or a functional fragment thereof or an antibody mimetic. Preferred antibodies of the invention are monoclonal antibodies.

The described affinity reagent may be a chimeric antibody, a human antibody, a humanized antibody, a single chain antibody, a defucosylated antibody.

Functional antibody fragments include is a UniBody, a domain antibody or a Nanobody.

Antibody mimetics include an Affibody, a DARPin, an Anticalin, an Avimer, a Versabody or a Duocalin.

The affinity reagents described may contain or be conjugated to a therapeutic moiety, such as a cytotoxic moiety or a radioactive isotope. The affinity reagent may be an antibody drug conjugate or immunoconjugate.

The described affinity reagent may elicit antibody-dependent cellular cytotoxicity (ADCC) or may elicit complement dependent cytotoxicity (CDC). The affinity reagent may induce apoptosis of cancer cells, kill or reduce the number of cancer stem cells and/or kill or reduce the number of circulating cancer cells. Affinity reagents may modulate a physiological function of NAALADL2, inhibit ligand binding to NAALADL2 and/or inhibit a signal transduction pathway mediated by NAALADL2.

Also described is a method for the treatment or prophylaxis of cancer wherein NAALADL2 is expressed in said cancer, which comprises administering to a subject in need thereof a therapeutically effective amount of a hybridizing agent capable of hybridizing to nucleic acid encoding NAALADL2.

The disclosure describes a hybridizing agent capable of hybridizing to nucleic acid encoding NAALADL2 for use in the treatment or prophylaxis of a cancer, preferably wherein the cancer is one of the diseases of the invention.

Also described is the use of a hybridizing agent capable of hybridizing to nucleic acid encoding NAALADL2 in the manufacture of a medicament for the treatment or prophylaxis of a cancer.

The hybridizing agents described preferably bind specifically to nucleic acid encoding one or more extracellular domains of NAALADL2.

Suitable hybridizing agents for use in the described methods include inhibitory RNA, short interfering RNA (siRNA), short hairpin RNA (shRNA), microRNA (miRNA), anti-sense nucleic acid, complementary DNA (cDNA), oligonucleotides and ribozymes.

Also disclosed is a method of detecting, diagnosing and/or screening for or monitoring the progression of a cancer wherein NAALADL2 is expressed in said cancer, or of monitoring the effect of a cancer drug or therapy wherein NAALADL2 is expressed in said cancer, in a subject which comprises detecting the presence or level of NAALADL2, or one or more fragments thereof, or the presence or level of nucleic acid encoding NAALADL2 or which comprises detecting a change in the level thereof in said subject.

Such a method may comprise detecting the presence of NAALADL2, or one or more fragments thereof, or the presence of nucleic acid encoding NAALADL2, in which either (a) the presence of an elevated level of NAALADL2 or said one or more fragments thereof or an elevated level of nucleic acid encoding NAALADL2 in the subject as compared with the level in a healthy subject, or (b) the presence of a detectable level of NAALADL2 or said one or more fragments thereof or a detectable level of nucleic acid encoding NAALADL2 in the subject as compared with a corresponding undetectable level in a healthy subject is indicative of the presence of the cancer wherein NAALADL2 is expressed in said cancer, in said subject.

The description also describes a method of detecting, diagnosing and/or screening for or monitoring the progression a cancer wherein NAALADL2 is expressed in said cancer, or of monitoring the effect of a cancer drug or therapy wherein NAALADL2 is expressed in said cancer, in a subject which comprises detecting the presence or level of antibodies capable of immunospecific binding to NAALADL2, or one or more fragments thereof.

In the methods described herein, the presence of NAALADL2, or one or more fragments thereof, or the presence of nucleic acid encoding NAALADL2, or the presence or level of antibodies capable of immunospecific binding to NAALADL2, or one or more fragments thereof, may be detected by analysis of a biological sample obtained from the subject.

The presence of NAALADL2, or one or more fragments thereof, may be detected using an affinity reagent which binds to NAALADL2. The affinity reagent may be any suitable affinity reagent as mentioned herein. The affinity reagent may contain or be conjugated to a detectable label.

In any of the aspects of the invention referred to herein, the subject may be a human.

The disclosure also describes methods for identifying an agent for the treatment or prophylaxis of cancer wherein NAALADL2 is expressed in said cancer, wherein the method comprises (a) contacting NAALADL2, or one or more fragments thereof, with a candidate agent; and (b) determining whether the agent binds to NAALADL2, or one or more fragments thereof. The method may also further comprise the step of testing the ability of an agent which binds to NAALADL2, or one or more fragments thereof, to inhibit cancer wherein NAALADL2 is expressed in said cancer. The agent may, *inter alia,* modulate an activity of NAALADL2, reduce ligand binding to NAALADL2 or reduce NAALADL2 dimerisation.

In the various example described herein, particular cancer types may be mentioned

In one example the cancer to be detected, prevented or treated is selected from prostate cancer, breast cancer, colorectal cancer, gastric cancer and pancreatic cancer.

In one embodiment the cancer to be treated is prostate cancer.

In another example the cancer to be detected, prevented or treated is bladder cancer.

In another example the cancer to be detected, prevented or treated is breast cancer, e.g. ER negative, PR negative and Her2 negative breast cancer, i.e. Triple negative breast cancer.

In another example the cancer to be detected, prevented or treated is esophageal cancer.

In another example the cancer to be detected, prevented or treated is head and neck cancer.

In another example the cancer to be detected, prevented or treated is colorectal cancer.

In another example the cancer to be detected, prevented or treated is liver cancer, e.g. hepatocellular carcinoma.

In another example the cancer to be detected, prevented or treated is lung cancer, e.g. non-small cell lung cancer and/or small cell lung cancer.

In another example the cancer to be detected, prevented or treated is ovarian cancer.

In another example the cancer to be detected, prevented or treated is gastric cancer.

In another example the cancer to be detected, prevented or treated is pancreatic cancer.

In another example the cancer to be detected, prevented or treated is uterus cancer.

Other aspects of the present invention are set out below and in the claims herein.

### BRIEF DESCRIPTION OF THE FIGURES

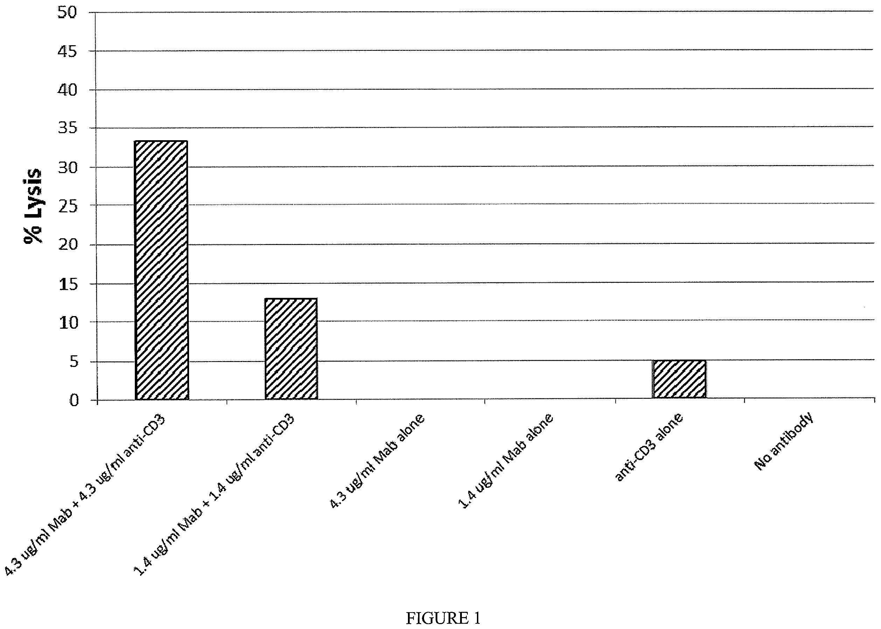

Figure 1 shows the specific lysis of LnCAP NAALADL2 expressing cells by activation of T cells by bispecific anti-NAALADL2-anti-CD3 polyclonal antibodies.

### DETAILED DESCRIPTION OF THE INVENTION

The disclosure described in detail below encompasses the administration of therapeutic compositions to a subject, e.g. a mammalian subject, to treat or prevent cancer, e.g. the diseases described herein. The description also provides methods and compositions for clinical screening, diagnosis and prognosis of cancer, e.g. the diseases of the description, in a mammalian subject for identifying patients most likely to respond to a particular therapeutic treatment, for monitoring the results of cancer e.g. the diseases of the description therapy, for drug screening and drug development.

The invention is based on the finding that NAALADL2 protein is expressed in certain cancers. In particular, supporting data is enclosed herein which demonstrates the expression of NAALADL2 protein in the plasma membrane of prostate cancer, breast cancer, colorectal cancer, gastric cancer and pancreatic cancer. Immuno-histochemical analysis also shows strong staining of prostate cancer, colorectal cancer and breast cancer, with more moderate staining in esophagus cancer, head and neck cancer, liver cancer, lung cancer, ovarian cancer, gastric cancer and uterus cancer. Therefore antibodies directed to NAALADL2 may have utility as therapeutics and diagnostics in these cancers and other cancer types showing expression of NAALADL2.

As used herein, the term "subject" refers to animal, preferably a mammal. The mammalian subject may be a non-human mammal, but is generally a human, such as a human adult.

The subject will in general be a living subject. However, whilst the uses, methods and compositions of the disclosure are especially suited for screening, diagnosis and prognosis of a living subject, they may also be used for postmortem diagnosis in a subject, for example, to identify family members at risk of developing the same disease.

As used herein, the term "patient" refers to a subject who has or is suspected of having one or more of the disclosed diseases.

As used herein, the term "protein of the invention" refers to Inactive N-acetylated-alpha-linked acidic dipeptidase-like protein 2 (GeneID: 254827), which is referred to herein as NAALADL2. This protein has been found to be differentially expressed in various cancers thus providing a new target for affinity-based therapies of these cancers. A human sequence of the NAALADL2 protein is given in SEQ ID NO: 1. The term NAALADL2 (in the context of a protein) encompasses proteins whose amino acid sequences consist of or comprise the amino acid sequence given in SEQ ID NO: 1 or derivatives or variants thereof, particularly naturally-occurring human derivatives or variants thereof.

This protein has been identified in membrane protein extracts of cancer tissue samples from cancer patients through the methods and apparatus described in Example 1 (e.g. by liquid chromatography-mass spectrometry of membrane protein extracts). Peptide sequences were compared to the SWISS PROT and TrEMBL databases (held by the Swiss Institute of Bioinformatics (SIB) and the European Bioinformatics Institute (EBI) which are available at www.expasy.org), and the entry Q58DX5, Inactive N-acetylated-alpha-linked acidic dipeptidase-like protein 2 - NAALADL2, was identified. The nucleotide sequence encoding this protein is found at accession number NM_207015, as given in SEQ ID NO: 3.

According to SWISS-PROT, NAALADL2 is single-pass protein of the peptidase M28 family. The protein consists of one transmembrane region and has an extracellular tail of 653 amino acids between amino acids 143-795 of SEQ ID NO: 1 (SEQ ID NO: 19). NAALADL2 has previously been disclosed in WO2009028521 where siRNA targeting PIKB and NAALADL2 genes were used for inhibiting cellular growth of prostate cancer cells. The inventor has shown NAALADL2 expression by immunohistochemistry on the surface of cancer cells and not normal prostate cells, suggesting affinity-based therapies directed against the NAALADL2 protein (SEQ ID NOs: 1-2) in patients, including those with these cancers, will have a therapeutic effect.

Immuno-histochemical analysis also shows strong staining of prostate cancer, colorectal cancer and breast cancer, with more moderate staining in esophagus cancer, head and neck cancer, liver cancer, lung cancer, ovarian cancer, gastric cancer and uterus cancer.

NAALADL2 is useful as are fragments particularly epitope containing fragments e.g. antigenic or immunogenic fragments thereof and derivatives thereof, particularly fragments comprising extracellular domains (e.g. extracellular tails or loops) of the protein. Epitope containing fragments, including antigenic or immunogenic fragments, will typically be of length 12 amino acids or more, e.g. 20 amino acids or more, e.g. 50 or 100 amino acids or more. Fragments may be 95% or more of the length of the full protein, e.g. 90% or more, e.g. 75% or 50% or 25% or 10% or more of the length of the full protein.

Alternatively, the protein/polypeptide employed or referred to herein may be limited to those proteins/polypeptides specifically recited/described in the present specification or to a variant or derivative which has at least 80, 85, 90, 91, 92, 93, 94, 95, 96, 97, 98 or 99% amino acid sequence identity or similarity thereto. Percentage amino acid sequence identity/similarity may be determined by any suitable algorithm, e.g. BLAST, CLUSTAL, using appropriate default parameters.

Hence the term "NAALADL2" in the context of a protein or polypeptide refers to a protein whose amino acid sequence consists of or comprises the amino sequence given in any of SEQ ID NOs: 1-2 or a derivative or variant thereof which has at least 90% or 95% sequence identity to any of SEQ ID NOs: 1-2 and which protein has essentially the same tissue distribution as NAALADL2.

In the context of a nucleic acid, the term "NAALADL2" refers to a nucleic acid whose nucleotide sequence encodes a protein comprising the amino sequence given in any of SEQ ID NOs: 1-2 or a derivative or variant thereof which has at least 90% or 95% sequence identity to any of SEQ ID NOs: 1-2 and which protein has essentially the same tissue distribution as NAALADL2 protein.

The term "NAALADL2" in the context of a nucleic acid also refers to a nucleic acid whose nucleotide sequence comprises the sequence given in any of SEQ ID NOs: 3-4 or a derivative or variant thereof which has at least 90% or 95% sequence identity to any of SEQ ID NOs: 3-4 and which encodes a protein which has essentially the same tissue distribution as NAALADL2 protein.

Epitope-containing fragments of NAALADL2 including antigenic or immunogenic fragments will be capable of eliciting a relevant immune response in a patient. DNA encoding NAALADL2 is also useful as are fragments thereof, e.g. DNA encoding fragments of NAALADL2 such as immunogenic fragments thereof. Fragments of nucleic acid (e.g. DNA) encoding NAALADL2 may be 95% or more of the length of the full coding region, e.g. 90% or more e.g. 75% or 50% or 25% or 10% or more of the length of the full coding region. Fragments of nucleic acid (e.g. DNA) may be 36 nucleotides or more, e.g. 60 nucleotides or more, e.g. 150 or 300 nucleotides or more in length.

Derivatives of NAALADL2 include variants on the sequence in which one or more (e.g. 1-20 such as 15 amino acids, or up to 20% such as up to 10% or 5% or 1% by number of amino acids based on the total length of the protein) deletions, insertions or substitutions have been made. Substitutions may typically be conservative substitutions. Derivatives will typically have essentially the same biological function as the protein from which they are derived. Derivatives will typically be comparably antigenic or immunogenic to the protein from which they are derived. Derivatives will typically have either the ligand-binding activity, or the active receptor-complex forming ability, or preferably both, of the protein from which they are derived. Derivatives and variants will generally have the same tissue distribution as NAALADL2.

Derivatives of proteins also include chemically treated protein such as carboxymethylated, carboxyamidated, acetylated proteins, for example treated during purification.

In one example the disclosure provides NAALADL2 or a composition comprising NAALADL2. The protein may be in isolated or purified form. The description further discloses a nucleic acid encoding NAALADL2 and a composition comprising a nucleic acid encoding NAALADL2.

In a further example, there is provided a composition capable of eliciting an immune response in a subject, which composition comprises a NAALADL2 polypeptide and/or one or more antigenic or immunogenic fragments thereof, and one or more suitable carriers, excipients, diluents or adjuvants (suitable adjuvants are discussed below).

The composition capable of eliciting an immune response may for example be provided as a vaccine comprising a NAALADL2 polypeptide or derivative or variant thereof, and/or one or more antigenic or immunogenic fragments thereof, optionally together with one or more suitable carriers, excipients, diluents or adjuvants.

In another example the disclosure provides a NAALADL2 polypeptide, or one or more fragments or derivatives or variants thereof, for the treatment or prophylaxis of e.g. one or more of the diseases of the disclosure.

In another example the disclosure provides a use of a NAALADL2 polypeptide, or one or more fragments or derivatives or variants thereof, for the treatment or prophylaxis of e.g. one or more of the disclosed diseases.

The description also provides a use of a NAALADL2 polypeptide, one or more fragments or derivatives or variants thereof, in the manufacture of a medicament for the treatment or prophylaxis of e.g. one or more of the disclosed diseases.

In one example there is provided a method of treatment comprising admininstering a therapeutically effective amount of a NAALADL2 polypeptide, one or more fragments or derivatives or variants thereof, for the treatment or prophylaxis of e.g. one or more of the disclosed diseases.

The description further provides a method for the treatment or prophylaxis of e.g. the disclosed diseases in a subject, or of vaccinating a subject against e.g. one or more of the disclosed diseases, which comprises the step of administering to the subject an effective amount of a NAALADL2 polypeptide and/or one or more antigenic or immunogenic fragments or derivatives or variants thereof, for example as a vaccine.

In another example the disclosure provides methods of treating e.g. the disclosed diseases, comprising administering to a patient a therapeutically effective amount of a compound that modulates (e.g. upregulates or downregulates) or complements the expression or the biological activity (or both) of NAALADL2 in patients having e.g. the disclosed diseases, in order to (a) prevent the onset or development of e.g. the disclosed diseases; (b) prevent the progression of e.g. the disclosed diseases; or (c) ameliorate the symptoms of e.g. the disclosed diseases.

In yet a further example, the disclosure provides a medicament comprising, separately or together:
(a) NAALADL2, and
(b) an anti-cancer agent,
for simultaneous, sequential or separate administration in the treatment of cancer, preferably in the treatment of one of the disclosed diseases.

NAALADL2 can be used for detection, prognosis, diagnosis, or monitoring of, e.g. the disclosed diseases or for drug development.

According to another example, we provide a method of detecting, diagnosing and/or screening for or monitoring the progression of e.g. the disclosed diseases or of monitoring the effect of e.g. an anti-cancer drug or therapy directed towards the disclosed diseases in a subject which comprises detecting the presence or level of NAALADL2, or one or more fragments thereof, or the presence or level of nucleic acid encoding NAALADL2 or the presence or level of the activity of NAALADL2 or which comprises detecting a change in the level thereof in said subject.

According to another example we provide a method of detecting, diagnosing and/or screening for e.g. the disclosed diseases in a candidate subject which comprises detecting the presence of NAALADL2, or one or more fragments thereof, or the presence of nucleic acid encoding NAALADL2 or the presence of the activity of NAALADL2 in said candidate subject, in which either (a) the presence of an elevated level of NAALADL2 or said one or more fragments thereof or an elevated level of nucleic acid encoding NAALADL2 or the presence of an elevated level of NAALADL2 activity in the candidate subject as compared with the level in a healthy subject or (b) the presence of a detectable level of NAALADL2 or said one or more fragments thereof or a detectable level of nucleic acid encoding NAALADL2 or the presence of a detectable level of NAALADL2 activity in the candidate subject as compared with a corresponding undetectable level in a healthy subject indicates the presence of e.g. the disclosed diseases.

According to example, we provide a method of monitoring the progression of e.g. the disclosed diseases in a subject or of monitoring the effect of e.g. an anti-cancer drug or therapy directed towards the disclosed diseases which comprises detecting the presence of NAALADL2, or one or more fragments thereof, or the presence of nucleic acid encoding NAALADL2 or the presence of the activity of NAALADL2 in said candidate subject at a first time point and at a later time point, the presence of an elevated or lowered level of NAALADL2 or said one or more fragments thereof or an elevated or lowered level of nucleic acid encoding NAALADL2 or the presence of an elevated or lowered level of NAALADL2 activity in the subject at the later time point as compared with the level in the subject at said first time point, indicating the progression or regression of e.g. the disclosed diseases or indicating the effect or non-effect of e.g. an anti-cancer drug or therapy directed towards the disclosed diseases in said subject.

For NAALADL2, the detected level obtained upon analyzing tissue sample from subjects having e.g. the disclosed diseases relative to the detected level obtained upon analyzing tissue from subjects free from e.g. the disclosed diseases will depend upon the particular analytical protocol and detection technique that is used. Accordingly, the disclosure contemplates that each laboratory will establish a reference range in subjects free from e.g. the disclosed diseases according to the analytical protocol and detection technique in use, as is conventional in the diagnostic art. Preferably, at least one control positive tissue sample from a subject known to have e.g. the disclosed diseases or at least one control negative tissue sample from a subject known to be free from e.g. the disclosed diseases (and more preferably both positive and negative control samples) are included in each batch of test samples analysed.

In one example, liquid chromatography-mass spectrometry analysis or other appropriate methods are used to analyze the tissue samples from a subject, preferably a living subject, in order to measure the expression of NAALADL2 for screening or diagnosis of e.g. the disclosed diseases, to determine the prognosis of a patient, to monitor the effectiveness of the therapy, or for drug development.

In any of the above methods, the level that may be detected in the candidate subject who has cancer, e.g. the disclosed diseases is preferably 2 or more fold higher than the level in the healthy subject.

In one example, tissue sample from a subject (e.g. a subject suspected of having the disclosed diseases) is analysed by liquid chromatography-mass spectrometry for detection of NAALADL2. An increased abundance of NAALADL2 in the tissue from the subject relative to tissue from a subject or subjects free from the disclosed diseases (e.g. a control sample) or a previously determined reference range indicates the presence of the disclosed diseases.

In relation to fragments, epitope containing fragments, immunogenic fragments or antigenic fragments of NAALADL2:
for the relevant cancer applications, in one example these comprise the sequence identified as a tryptic sequence in Example 1.

As used herein, NAALADL2 is "isolated" when it is present in a preparation that is substantially free of contaminating proteins, i.e. a preparation in which less than 10% (for example less than 5%, such as less than 1%) of the total protein present is contaminating protein(s). A contaminating protein is a protein having a significantly different amino acid sequence from that of isolated NAALADL2, as determined by mass spectral analysis. As used herein, a "significantly different" sequence is one that permits the contaminating protein to be resolved from NAALADL2 by mass spectral analysis, performed according to the protocol described herein in Example 1.

In the diagnostic and prognostic methods described, NAALADL2 can be assayed by any method known to those skilled in the art, including but not limited to, the Preferred Technologies described herein, kinase assays, enzyme assays, binding assays and other functional assays, immunoassays, and western blotting.

Alternatively, NAALADL2 can be detected in an immunoassay. In one example, an immunoassay is performed by contacting a sample from a subject to be tested with an anti-NAALADL2 antibody (or other affinity reagent) under conditions such that binding (e.g. immunospecific binding) can occur if NAALADL2 is present, and detecting or measuring the amount of any binding (e.g. immunospecific binding) by the agent. NAALADL2 binding agents can be produced by the methods and techniques taught herein. In a particular example, NAALADL2 is analysed using immunohistochemistry.

NAALADL2 may be detected by virtue of the detection of a fragment thereof e.g. an epitope containing (e.g. an immunogenic or antigenic) fragment thereof. Fragments may have a length of at least 10, more typically at least 20 amino acids e.g. at least 50 or 100 amino acids e.g. at least 150 or 200 amino acids; e.g. at least 300 or 500 amino acids; e.g. at least 700 or 900 amino acids.

In one example, binding of an affinity reagent (e.g. an antibody) in tissue sections can be used to detect aberrant NAALADL2 localization or an aberrant level of NAALADL2. In one example, an antibody (or other affinity reagent) to NAALADL2 can be used to assay a patient tissue (e.g. a prostate, bladder, breast, esophagus, head and neck, colorectal, liver, lung, ovarian, gastric, uterus and pancreatic tissue) for the level of NAALADL2 where an aberrant level of NAALADL2 is indicative of the disclosed diseases. As used herein, an "aberrant level" means a level that is increased compared with the level in a subject free from the disclosed diseases or a reference level.

Any suitable immunoassay can be used, including, without limitation, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

For example, NAALADL2 can be detected in a fluid sample (e.g. blood, urine, or saliva) by means of a two-step sandwich assay. In the first step, a capture reagent (e.g. an anti-NAALADL2 antibody or other affinity reagent) is used to capture NAALADL2. The capture reagent can optionally be immobilized on a solid phase. In the second step, a directly or indirectly labeled detection reagent is used to detect the captured NAALADL2. In one example, the detection reagent is a lectin. Any lectin can be used for this purpose that preferentially binds to NAALADL2 rather than to other isoforms that have the same core protein as NAALADL2 or to other proteins that share the antigenic determinant recognized by the antibody. In a preferred embodiment, the chosen lectin binds NAALADL2 with at least 2-fold greater affinity, more preferably at least 5-fold greater affinity, still more preferably at least 10-fold greater affinity, than to said other isoforms that have the same core protein as NAALADL2 or to said other proteins that share the antigenic determinant recognized by the affinity reagent. Based on the present description, a lectin that is suitable for detecting NAALADL2 can readily be identified by methods well known in the art, for instance upon testing one or more lectins enumerated in Table I on pages 158-159 of Sumar et al., Lectins as Indicators of Disease-Associated Glycoforms, In: Gabius H-J & Gabius S (eds.), 1993, Lectins and Glycobiology, at pp. 158-174. In a further example, the detection reagent is an antibody (or other affinity reagent), e.g. an antibody that specifically (e.g. immunospecifically) detects other post-translational modifications, such as an antibody that immunospecifically binds to phosphorylated amino acids. Examples of such antibodies include those that bind to phosphotyrosine (BD Transduction Laboratories, catalog nos.: P11230-050/P11230-150; P11120; P38820; P39020), those that bind to phosphoserine (Zymed Laboratories Inc., South San Francisco, CA, catalog no. 61-8100) and those that bind to phosphothreonine (Zymed Laboratories Inc., South San Francisco, CA, catalogue nos. 71-8200, 13-9200).

If desired, a gene encoding NAALADL2, a related gene, or related nucleic acid sequences or subsequences, including complementary sequences, can also be used in hybridization assays. A nucleotide encoding NAALADL2, or subsequences thereof comprising at least 8 nucleotides, preferably at least 12 nucleotides, and most preferably at least 15 nucleotides can be used as a hybridization probe. Hybridization assays can be used for detection, prognosis, diagnosis, or monitoring of conditions, disorders, or disease states, associated with aberrant expression of the gene encoding NAALADL2, or for differential diagnosis of subjects with signs or symptoms suggestive of e.g. the disclosed diseases. In particular, such a hybridization assay can be carried out by a method comprising contacting a subject's sample containing nucleic acid with a nucleic acid probe capable of hybridizing to a DNA or RNA that encodes NAALADL2, under conditions such that hybridization can occur, and detecting or measuring any resulting hybridization.

Hence nucleic acid encoding NAALADL2 (e.g. DNA or more suitably RNA) may be detected, for example, using a hybridizing agent (particularly an oligonucleotide probe) capable of hybridizing to nucleic acid encoding NAALADL2.

One such exemplary method comprises:
contacting one or more oligonucleotide probes comprising 10 or more consecutive nucleotides complementary to a nucleotide sequence encoding NAALADL2, with an RNA obtained from a biological sample from the subject or with cDNA copied from the RNA, wherein said contacting occurs under conditions that permit hybridization of the probe to the nucleotide sequence if present;
detecting hybridization, if any, between the probe and the nucleotide sequence; and
comparing the hybridization, if any, detected in step (b) with the hybridization detected in a control sample, or with a previously determined reference range.

The description also provides diagnostic kits, comprising an anti-NAALADL2 antibody (or other affinity reagent). In addition, such a kit may optionally comprise one or more of the following:
(1) instructions for using the anti-NAALADL2 affinity reagent for diagnosis, prognosis, therapeutic monitoring or any combination of these applications;
(2) a labeled binding partner to the affinity reagent;
(3) a solid phase (such as a reagent strip) upon which the anti-NAALADL2 affinity reagent is immobilized; and
(4) a label or insert indicating regulatory approval for diagnostic, prognostic or therapeutic use or any combination thereof. If no labeled binding partner to the affinity reagent is provided, the anti-NAALADL2 affinity reagent itself can be labeled with a detectable marker, e.g. a chemiluminescent, enzymatic, fluorescent, or radioactive moiety.

The description also provides a kit comprising a nucleic acid probe capable of hybridizing to nucleic acid, suitably RNA, encoding NAALADL2. In a specific example, a kit comprises one or more containers a pair of primers (e.g. each in the size range of 6-30 nucleotides, more preferably 10-30 nucleotides and still more preferably 10-20 nucleotides) that under appropriate reaction conditions can prime amplification of at least a portion of a nucleic acid encoding NAALADL2, such as by polymerase chain reaction (see, e.g. Innis et al., 1990, PCR Protocols, Academic Press, Inc., San Diego, CA), ligase chain reaction (see EP 320,308) use of Qβ replicase, cyclic probe reaction, or other methods known in the art.

A kit can optionally further comprise a predetermined amount of NAALADL2 or a nucleic acid encoding NAALADL2, e.g. for use as a standard or control.

As used herein, the term "sample" includes a bodily fluid (e.g. blood, urine or saliva) and tissue biopsies taken from a subject at risk of having one or more of the disclosed diseases (e.g. a biopsy such as a prostate, bladder, breast, esophagus, head and neck, colorectal, liver, lung, ovarian, gastric, uterus and pancreatic biopsy) or homogenate thereof.

For example, the biological sample used can be from any source such as a serum sample or a tissue sample e.g. prostate, bladder, breast, esophagus, head and neck, colorectal, liver, lung, ovarian, gastric, uterus and pancreatic tissue. For instance, when looking for evidence of metastatic the disclosed diseases, one would look at major sites of the disclosed diseases metastasis, e.g. the bones, bladder, rectum for prostate cancer, the bones, lungs, skin and the liver for bladder cancer, the bones, liver and lungs for breast cancer, the liver, peritoneal cavity, pelvis, retroperitoneum and lungs for colorectal cancer, the lungs, bones, liver and skin for head and neck cancer, the lungs and bone for liver cancer, the brain, liver, bones and adrenal glands for lung cancer, the liver for pancreatic cancer, the abdomen for ovarian cancer, the liver, lungs and bones for esophageal cancer, the bladder, rectum, lungs and bones for uterine cancer and the liver, lungs, brain, bones, kidneys and pancreas for gastric cancer.

Alternatively the presence of NAALADL2, or one or more fragments thereof, or the presence of nucleic acid encoding NAALADL2 or the presence of the activity of NAALADL2 may be detected by analysis in situ.

In certain examples, methods of diagnosis described herein may be at least partly, or wholly, performed *in vitro* or *ex vivo.*

Suitably the presence of NAALADL2, or one or more fragments thereof, or the presence of nucleic acid encoding NAALADL2 or the presence of the activity of NAALADL2 is detected quantitatively.

For example, quantitatively detecting may comprise:
contacting a biological sample with an affinity reagent that is specific for NAALADL2, said affinity reagent optionally being conjugated to a detectable label; and
detecting whether binding has occurred between the affinity reagent and at least one species in the sample, said detection being performed either directly or indirectly.

Alternatively the presence of NAALADL2, or one or more fragments thereof, or the presence of nucleic acid encoding NAALADL2 or the presence of the activity of NAALADL2 may be detected quantitatively by means involving use of an imaging technology.

In another example, the method involves use of immunohistochemistry on e.g. prostate, bladder, breast, esophagus, head and neck, colorectal, liver, lung, ovarian, gastric, uterus and pancreatic tissue sections in order to determine the presence of NAALADL2, or one or more fragments thereof, or the presence of nucleic acid encoding NAALADL2 or the presence of the activity of NAALADL2, and thereby to localise e.g. the disclosed diseases cells.

In one example the presence of NAALADL2 or one or more epitope-containing fragments thereof is detected, for example using an affinity reagent capable of specific binding to NAALADL2 or one or more fragments thereof, such as an antibody.

In another example the activity of NAALADL2 is detected.

### Use in Clinical Studies

The diagnostic methods and compositions of the disclosure can assist in monitoring a clinical study, e.g. to evaluate drugs for therapy of the disclosed diseases. In one example, candidate molecules are tested for their ability to restore NAALADL2 levels in a subject having e.g. the disclosed diseases to levels found in subjects free from the disclosed diseases or, in a treated subject, to preserve NAALADL2 levels at or near non-prostate cancer, non-bladder cancer, non-breast cancer, non-esophagus cancer, non-head and neck cancer, non-colorectal cancer, non-liver cancer, non-lung cancer, non-ovarian cancer, non-gastric cancer, non-uterus cancer or non-pancreatic cancer values.

In another example, the methods and compositions of the disclosure are used to screen candidates for a clinical study to identify individuals having e.g. the disclosed diseases; such individuals can then be excluded from the study or can be placed in a separate cohort for treatment or analysis.

### Production of Protein of the and Corresponding Nucleic Acid

In one example the disclosure provides a method of treating or preventing e.g. the disclosed diseases, comprising administering to a subject in need of such treatment or prevention a therapeutically effective amount of nucleic acid encoding NAALADL2 or one or more fragments or derivatives thereof, for example in the form of a vaccine.

In another example there is provided a method of treating or preventing e.g. the disclosed diseases comprising administering to a subject in need of such treatment or prevention a therapeutically effective amount of nucleic acid that inhibits the function or expression of NAALADL2.

The methods (and/or other DNA examples disclosed herein) of the description may, for example include wherein the nucleic acid is a NAALADL2 anti-sense nucleic acid or ribozyme.

Thus the description includes the use of nucleic acid encoding NAALADL2 or one or more fragments or derivatives thereof, in the manufacture of a medicament for treating or preventing e.g. the disclosed diseases.

There is also provided the use of nucleic acid that inhibits the function or expression of NAALADL2 in the manufacture of a medicament for treating or preventing e.g. one or more of the disclosed diseases.

A DNA employed in the present disclosure can be obtained by isolation as a cDNA fragment from cDNA libraries using as starter materials commercial mRNAs and determining and identifying the nucleotide sequences thereof. That is, specifically, clones are randomly isolated from cDNA libraries, which are prepared according to Ohara et al.'s method (DNA Research Vol.4, 53-59 (1997)). Next, through hybridization, duplicated clones (which appear repeatedly) are removed and then *in vitro* transcription and translation are carried out. Nucleotide sequences of both termini of clones, for which products of 50 kDa or more are confirmed, are determined.

Furthermore, databases of known genes are searched for homology using the thus obtained terminal nucleotide sequences as queries.

In addition to the above screening method, the 5' and 3' terminal sequences of cDNA are related to a human genome sequence. Then an unknown long-chain gene is confirmed in a region between the sequences, and the full-length of the cDNA is analyzed. In this way, an unknown gene that is unable to be obtained by a conventional cloning method that depends on known genes can be systematically cloned.

Moreover, all of the regions of a human-derived gene containing a DNA of the present description can also be prepared using a PCR method such as RACE while paying sufficient attention to prevent artificial errors from taking place in short fragments or obtained sequences. As described above, clones having DNA of the present description can be obtained.

In another means for cloning DNA of the present description, a synthetic DNA primer having an appropriate nucleotide sequence of a portion of a polypeptide of the present description is produced, followed by amplification by the PCR method using an appropriate library. Alternatively, selection can be carried out by hybridization of the DNA of the present description with a DNA that has been incorporated into an appropriate vector and labeled with a DNA fragment or a synthetic DNA encoding some or all of the regions of the polypeptide of the present description. Hybridization can be carried out by, for example, the method described in Current Protocols in Molecular Biology (edited by Frederick M. Ausubel et al., 1987). DNA of the present description may be any DNA, as long as they contain nucleotide sequences encoding the polypeptides of the present description as described above. Such a DNA may be a cDNA identified and isolated from cDNA libraries or the like that are derived from prostate, bladder, breast, esophagus, head and neck, colorectal, liver, lung, ovarian, gastric, uterus and pancreatic tissue. Such a DNA may also be a synthetic DNA or the like. Vectors for use in library construction may be any of bacteriophages, plasmids, cosmids, phargemids, or the like. Furthermore, by the use of a total RNA fraction or a mRNA fraction prepared from the above cells and/or tissues, amplification can be carried out by a direct reverse transcription coupled polymerase chain reaction (hereinafter abbreviated as "RT-PCR method").

DNA encoding the above polypeptide consisting of an amino acid sequence that is substantially identical to the amino acid sequence of NAALADL2 or DNA encoding the above polypeptide consisting of an amino acid sequence derived from the amino acid sequence of NAALADL2 by deletion, substitution, or addition of one or more amino acids composing a portion of the amino acid sequence can be easily produced by an appropriate combination of, for example, a site-directed mutagenesis method, a gene homologous recombination method, a primer elongation method, and the PCR method known by persons skilled in the art. In addition, at this time, a possible method for causing a polypeptide to have substantially equivalent biological activity is substitution of homologous amino acids (e.g. polar and nonpolar amino acids, hydrophobic and hydrophilic amino acids, positively-charged and negatively charged amino acids, and aromatic amino acids) among amino acids composing the polypeptide. Furthermore, to maintain substantially equivalent biological activity, amino acids within functional domains contained in the polypeptide of the present description are preferably conserved.

Furthermore, examples of DNA of the present description include DNA comprising a nucleotide sequence that encodes the amino acid sequence of NAALADL2 and DNA hybridizing under stringent conditions to the DNA and encoding a polypeptide (protein) having biological activity (function) equivalent to the function of the polypeptide consisting of the amino acid sequence of NAALADL2. Under such conditions, an example of such DNA capable of hybridizing to DNA comprising the nucleotide sequence that encodes the amino acid sequence of NAALADL2 is DNA comprising a nucleotide sequence that has a degree of overall mean homology with the entire nucleotide sequence of the DNA, such as approximately 80% or more, preferably approximately 90% or more, and more preferably approximately 95% or more. Hybridization can be carried out according to a method known in the art such as a method described in Current Protocols in Molecular Biology (edited by Frederick M. Ausubel et al., 1987) or a method according thereto. Here, "stringent conditions" are, for example, conditions of approximately "1^{∗}SSC, 0.1% SDS, and 37°C, more stringent conditions of approximately "0.5^{∗}SSC, 0.1% SDS, and 42°C, or even more stringent conditions of approximately "0.2^{∗}SSC, 0.1% SDS, and 65°C. With more stringent hybridization conditions, the isolation of a DNA having high homology with a probe sequence can be expected. The above combinations of SSC, SDS, and temperature conditions are given for illustrative purposes. Stringency similar to the above can be achieved by persons skilled in the art using an appropriate combination of the above factors or other factors (for example, probe concentration, probe length, and reaction time for hybridization) for determination of hybridization stringency.

A cloned DNA of the present description can be directly used or used, if desired, after digestion with a restriction enzyme or addition of a linker, depending on purposes. The DNA may have ATG as a translation initiation codon at the 5' terminal side and have TAA, TGA, or TAG as a translation termination codon at the 3' terminal side. These translation initiation and translation termination codons can also be added using an appropriate synthetic DNA adapter.

In the methods/uses of the description, NAALADL2 may for example be provided in isolated form, such as where the NAALADL2 polypeptide has been purified to at least to some extent. NAALADL2 polypeptide may be provided in substantially pure form, that is to say free, to a substantial extent, from other proteins. NAALADL2 polypeptide can also be produced using recombinant methods, synthetically produced or produced by a combination of these methods. NAALADL2 can be easily prepared by any method known by persons skilled in the art, which involves producing an expression vector containing appropriate DNA of the present description or a gene containing a DNA of the present description, culturing a transformant transformed using the expression vector, generating and accumulating a relevant polypeptide of the present description or a recombinant protein containing the polypeptide, and then collecting the resultant.

Recombinant NAALADL2 polypeptide may be prepared by processes well known in the art from genetically engineered host cells comprising expression systems. Accordingly, the present description also relates to expression systems which comprise a NAALADL2 polypeptide or nucleic acid, to host cells which are genetically engineered with such expression systems and to the production of NAALADL2 polypeptide by recombinant techniques. For recombinant NAALADL2 polypeptide production, host cells can be genetically engineered to incorporate expression systems or portions thereof for nucleic acids. Such incorporation can be performed using methods well known in the art, such as, calcium phosphate transfection, DEAD-dextran mediated transfection, transvection, microinjection, cationic lipid-mediated transfection, electroporation, transduction, scrape loading, ballistic introduction or infection (see e.g. Davis et al., Basic Methods in Molecular Biology, 1986 and Sambrook et al., Molecular Cloning: A Laboratory Manual, 2nd Ed., Cold Spring Harbour laboratory Press, Cold Spring Harbour, NY, 1989).

As host cells, for example, bacteria of the genus *Escherichia, Streptococci, Staphylococci, Streptomyces,* bacteria of the genus *Bacillus,* yeast, *Aspergillus* cells, insect cells, insects, and animal cells are used. Specific examples of bacteria of the genus *Escherichia,* which are used herein, include *Escherichia coli* K12 and DH1 (Proc. Natl. Acad. Sci. U.S.A., Vol. 60, 160 (1968)), JM103 (Nucleic Acids Research, Vol. 9, 309 (1981)), JA221 (Journal of Molecular Biology, Vol. 120, 517 (1978)), and HB101 (Journal of Molecular Biology, Vol. 41, 459 (1969)). As bacteria of the genus Bacillus, for example, Bacillus subtilis MI114 (Gene, Vol. 24, 255 (1983)) and 207-21 (Journal of Biochemistry, Vol. 95, 87 (1984)) are used. As yeast, for example, *Saccaromyces cerevisiae* AH22, AH22R-, NA87-11A, DKD-5D, and 20B-12, *Schizosaccaromyces pombe* NCYC1913 and NCYC2036, and *Pichia pastoris* are used. As insect cells, for example, Drosophila S2 and Spodoptera Sf9 cells are used. As animal cells, for example, COS-7 and Vero monkey cells, CHO Chinese hamster cells (hereinafter abbreviated as CHO cells), dhfr-gene-deficient CHO cells, mouse L cells, mouse AtT-20 cells, mouse myeloma cells, rat GH3 cells, human FL cells, COS, HeLa, C127,3T3, HEK 293, BHK and Bowes melanoma cells are used.

Cell-free translation systems can also be employed to produce recombinant polypeptides (e.g. rabbit reticulocyte lysate, wheat germ lysate, SP6/T7 in vitro T&T and RTS 100 *E. Coli* HY transcription and translation kits from Roche Diagnostics Ltd., Lewes, UK and the TNT Quick coupled Transcription/Translation System from Promega UK, Southampton, UK).

The expression vector can be produced according to a method known in the art. For example, the vector can be produced by (1) excising a DNA fragment containing a DNA of the present description or a gene containing a DNA of the present description and (2) ligating the DNA fragment downstream of the promoter in an appropriate expression vector. A wide variety of expression systems can be used, such as and without limitation, chromosomal, episomal and virus-derived systems, e.g. plasmids derived from Escherichia coli (e.g. pBR322, pBR325, pUC18, and pUC118), plasmids derived from Bacillus subtilis (e.g. pUB110, pTP5, and pC194), from bacteriophage, from transposons, from yeast episomes (e.g. pSH19 and pSH15), from insertion elements, from yeast chromosomal elements, from viruses such as baculoviruses, papova viruses such as SV40, vaccinia viruses, adenoviruses, fowl pox viruses, pseudorabies viruses and retroviruses, and vectors derived from combinations thereof, such as those derived from plasmid and bacteriophage (such as [lambda] phage) genetic elements, such as cosmids and phagemids. The expression systems may contain control regions that regulate as well as engender expression. Promoters to be used in the present description may be any promoters as long as they are appropriate for hosts to be used for gene expression. For example, when a host is *Escherichia coli,* a trp promoter, a lac promoter, a recA promoter, a pL promoter, an lpp promoter, and the like are preferred. When a host is *Bacillus subtilis,* an SPO1 promoter, an SPO2 promoter, a penP promoter, and the like are preferred. When a host is yeast, a PHO5 promoter, a PGK promoter, a GAP promoter, an ADH promoter, and the like are preferred. When an animal cell is used as a host, examples of promoters for use in this case include an SRa promoter, an SV40 promoter, an LTR promoter, a CMV promoter, and an HSV-TK promoter. Generally, any system or vector that is able to maintain, propagate or express a nucleic acid to produce a polypeptide in a host may be used.

The appropriate nucleic acid sequence may be inserted into an expression system by any variety of well known and routine techniques, such as those set forth in Sambrook *et al.,* supra. Appropriate secretion signals may be incorporated into the NAALADL2 polypeptide to allow secretion of the translated protein into the lumen of the endoplasmic reticulum, the periplasmic space or the extracellular environment. These signals may be endogenous to the NAALADL2 polypeptide or they may be heterologous signals. Transformation of the host cells can be carried out according to methods known in the art. For example, the following documents can be referred to: Proc. Natl. Acad. Sci. U.S.A., Vol. 69, 2110 (1972); Gene, Vol. 17, 107 (1982); Molecular & General Genetics, Vol. 168, 111 (1979); Methods in Enzymology, Vol. 194, 182-187 (1991); Proc. Natl. Acad. Sci. U.S.A.), Vol. 75, 1929 (1978); Cell Technology, separate volume 8, New Cell Technology, Experimental Protocol. 263-267 (1995) (issued by Shujunsha); and Virology, Vol. 52, 456 (1973). The thus obtained transformant transformed with an expression vector containing a DNA of the present description or a gene containing a DNA of the present description can be cultured according to a method known in the art. For example, when hosts are bacteria of the genus *Escherichia,* the bacteria are generally cultured at approximately 15°C to 43°C for approximately 3 to 24 h. If necessary, aeration or agitation can also be added. When hosts are bacteria of the genus *Bacillus,* the bacteria are generally cultured at approximately 30°C to 40°C for approximately 6 to 24 h. If necessary, aeration or agitation can also be added. When transformants whose hosts are yeast are cultured, culture is generally carried out at approximately 20°C to 35°C for approximately 24 to 72 h using media with pH adjusted to be approximately 5 to 8. If necessary, aeration or agitation can also be added. When transformants whose hosts are animal cells are cultured, the cells are generally cultured at approximately 30°C to 40°C for approximately 15 to 60 h using media with the pH adjusted to be approximately 6 to 8. If necessary, aeration or agitation can also be added.

If a NAALADL2 polypeptide is to be expressed for use in cell-based screening assays, it is preferred that the polypeptide be produced at the cell surface. In this event, the cells may be harvested prior to use in the screening assay. If the NAALADL2 polypeptide is secreted into the medium, the medium can be recovered in order to isolate said polypeptide. If produced intracellularly, the cells must first be lysed before the NAALADL2 polypeptide is recovered.

NAALADL2 polypeptide can be recovered and purified from recombinant cell cultures or from other biological sources by well known methods including, ammonium sulphate or ethanol precipitation, acid extraction, anion or cation exchange chromatography, phosphocellulose chromatography, affinity chromatography, hydrophobic interaction chromatography, hydroxylapatite chromatography, molecular sieving chromatography, centrifugation methods, electrophoresis methods and lectin chromatography. In one example, a combination of these methods is used. In another example, high performance liquid chromatography is used. In a further example, an antibody which specifically binds to a NAALADL2 polypeptide can be used to deplete a sample comprising a NAALADL2 polypeptide of said polypeptide or to purify said polypeptide.

To separate and purify a polypeptide or a protein of the present description from the culture products, for example, after culture, microbial bodies or cells are collected by a known method, they are suspended in an appropriate buffer, the microbial bodies or the cells are disrupted by, for example, ultrasonic waves, lysozymes, and/or freeze-thawing, the resultant is then subjected to centrifugation or filtration, and then a crude extract of the protein can be obtained. The buffer may also contain a protein denaturation agent such as urea or guanidine hydrochloride or a surfactant such as Triton X-100(TM). When the protein is secreted in a culture solution, microbial bodies or cells and a supernatant are separated by a known method after the completion of culture and then the supernatant is collected. The protein contained in the thus obtained culture supernatant or the extract can be purified by an appropriate combination of known separation and purification methods. The thus obtained polypeptide (protein) of the present description can be converted into a salt by a known method or a method according thereto. Conversely, when the polypeptide (protein) of the present description is obtained in the form of a salt, it can be converted into a free protein or peptide or another salt by a known method or a method according thereto. Moreover, an appropriate protein modification enzyme such as trypsin or chymotrypsin is caused to act on a protein produced by a recombinant before or after purification, so that modification can be arbitrarily added or a polypeptide can be partially removed. The presence of a polypeptide (protein) of the present description or a salt thereof can be measured by various binding assays, enzyme immunoassays using specific antibodies, and the like.

Techniques well known in the art may be used for refolding to regenerate native or active conformations of the NAALADL2 polypeptide when the polypeptide has been denatured during isolation and or purification. In the context of the present disclosure, NAALADL2 polypeptide can be obtained from a biological sample from any source, such as and without limitation, a blood sample or tissue sample, e.g. a prostate, bladder, breast, esophagus, head and neck, colorectal, liver, lung, ovarian, gastric, uterus and pancreatic tissue sample.

NAALADL2 polypeptide may be in the form of a "mature protein" or may be part of a larger protein such as a fusion protein. It is often advantageous to include an additional amino acid sequence which contains secretory or leader sequences, a pre-, pro- or prepro-protein sequence, or a sequence which aids in purification such as an affinity tag, for example, but without limitation, multiple histidine residues, a FLAG tag, HA tag or myc tag.

NAALADL2 may, for example, be fused with a heterologous fusion partner such as the surface protein, known as protein D from Haemophilus Influenza B, a non-structural protein from influenzae virus such as NS1, the S antigen from Hepatitis B or a protein known as LYTA such as the C terminal thereof.

An additional sequence that may provide stability during recombinant production may also be used. Such sequences may be optionally removed as required by incorporating a cleavable sequence as an additional sequence or part thereof. Thus, a NAALADL2 polypeptide may be fused to other moieties including other polypeptides or proteins (for example, glutathione S-transferase and protein A). Such a fusion protein can be cleaved using an appropriate protease, and then separated into each protein. Such additional sequences and affinity tags are well known in the art. In addition to the above, features known in the art, such as an enhancer, a splicing signal, a polyA addition signal, a selection marker, and an SV40 replication origin can be added to an expression vector, if desired.

In one example there is provided an agent capable of specific binding to NAALADL2, or a fragment thereof, or a hybridising agent capable of hybridizing to nucleic acid encoding NAALADL2 or an agent capable of detecting the activity of NAALADL2 for use in treating, screening for, detecting and/or diagnosing disease, such as cancer, and especially the disclosed diseases.

### Production of Affinity Reagents to NAALADL2

In one example the description provides an affinity or immunoaffinity reagent which is capable of specific binding to NAALADL2 or a fragment thereof, for example an affinity reagent which contains or is conjugated to a detectable label or contains or is conjugated to a therapeutic moiety, such as a cytotoxic moiety. The affinity agent may, for example, be an antibody. The affinity reagent may be an isolated affinity reagent or a purified affinity reagent.

The affinity reagent for use in the disclosure may bind to an epitope on NAALADL2, e.g. one or more of the portions of any of SEQ ID NO: 1-2. Preferably, the affinity reagent specifically binds to the extracellular domain (e.g. the extracellular tail or extracellular loop) of NAALADL2 (e.g. to SEQ ID NO: 19).

According to those in the art, there are three main types of immunoaffinity reagent - monoclonal antibodies, phage display antibodies and smaller antibody-derived molecules such as Affibodies, Domain Antibodies (dAbs), Nanobodies, UniBodies, DARPins, Anticalins, Duocalins, Avimers or Versabodies. In general in applications according to the disclosure where the use of antibodies is stated, other affinity reagents (e.g. Affibodies, Domain Antibodies, Nanobodies, UniBodies, DARPins, Anticalins, Duocalins, Avimers or Versabodies) may be employed. Such substances may be said to be capable of immunospecific binding to NAALADL2. Where appropriate the term "affinity agent" shall be construed to embrace immunoaffinity reagents and other substances capable of specific binding to NAALADL2 including but not limited to ligands, lectins, streptavidins, antibody mimetics and synthetic binding agents.

### Production of Antibodies to NAALADL2

According to the disclosure NAALADL2, a NAALADL2 analog, a NAALADL2-related protein or a fragment or derivative of any of the foregoing may be used as an immunogen to generate antibodies which immunospecifically bind such an immunogen. Such immunogens can be isolated by any convenient means, including the methods described above. The term "antibody" as used herein refers to a peptide or polypeptide derived from, modeled after or substantially encoded by an immunoglobulin gene or immunoglobulin genes, or fragments thereof, capable of specifically binding an antigen or epitope. See, e.g. Fundamental Immunology, 3rd Edition, W.E. Paul, ed., Raven Press, N.Y. (1993); Wilson (1994) J. Immunol. Methods 175:267-273; Yarmush (1992) J. Biochem. Biophys. Methods 25:85-97. The term antibody includes antigen-binding portions, i.e., "antigen binding sites" (e.g. fragments, subsequences, complementarity determining regions (CDRs)) that retain capacity to bind antigen, including (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody". Bispecific antibodies of the invention include, but are not limited to polyclonal, monoclonal, humanized or chimeric antibodies, single chain antibodies, Fab fragments and F(ab')₂ fragments, fragments produced by a Fab expression library, anti-idiotypic (anti-Id) antibodies, and epitope-binding fragments of any of the above. The immunoglobulin molecules of the invention can be of any class (e.g. IgG, IgE, IgM, IgD and IgA such as IgG) or subclass of immunoglobulin molecule.

The term "specifically binds" or "binds specifically" (or "immunospecifically binds") is not intended to indicate that an antibody binds exclusively to its intended target. Rather, an antibody "specifically binds" if its affinity for its intended target is typically about 5-fold greater when compared to its affinity for a non-target molecule. Suitably there is no significant cross-reaction or cross-binding with undesired substances, especially naturally occurring proteins or tissues of a healthy person or animal. Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In some embodiments, specific binding between an antibody or other binding agent and an antigen means a binding affinity of at least 10⁶ M⁻¹. Antibodies may, for example, bind with affinities of at least about 10⁷ M⁻¹, and preferably between about 10⁸ M⁻¹ to about 10⁹ M⁻¹, about 10⁹ M⁻¹ to about 10¹⁰ M⁻¹, or about 10¹⁰ M⁻¹ to about 10¹¹ M⁻¹.

Affinity is calculated as K_{d} =k_{off} /kₒₙ (k_{off} is the dissociation rate constant, kₒₙ is the association rate constant and K_{d} is the equilibrium constant. Affinity can be determined at equilibrium by measuring the fraction bound (r) of labeled ligand at various concentrations (c). The data are graphed using the Scatchard equation: r/c = K(n-r):
where
r = moles of bound ligand/mole of receptor at equilibrium;
c = free ligand concentration at equilibrium;
K = equilibrium association constant; and
n = number of ligand binding sites per receptor molecule
By graphical analysis, r/c is plotted on the Y-axis versus r on the X-axis thus producing a Scatchard plot. The affinity is the negative slope of the line. k_{off} can be determined by competing bound labeled ligand with unlabeled excess ligand (see, e.g. U.S. Pat No. 6,316,409). The affinity of a targeting agent for its target molecule is for example at least about 1 x 10⁻⁶ moles/liter, such as at least about 1 x 10⁻⁷ moles/liter, such as at least about 1 x 10⁻⁸ moles/liter, especially at least about 1 x 10⁻⁹ moles/liter, and particularly at least about 1 x 10⁻¹⁰ moles/liter. Antibody affinity measurement by Scatchard analysis is well known in the art, see, e.g. van Erp et al., J. Immunoassay 12: 425-43, 1991; Nelson and Griswold, Comput. Methods Programs Biomed. 27: 65-8, 1988.

In one embodiment, any publicly available antibodies that recognize gene products of genes encoding NAALADL2 may be used. In another embodiment, methods known to those skilled in the art are used to produce antibodies that recognize NAALADL2, a NAALADL2 analog, a NAALADL2-related polypeptide, or a fragment or derivative of any of the foregoing. One skilled in the art will recognize that many procedures are available for the production of antibodies, for example, as described in Antibodies, A Laboratory Manual, Ed Harlow and David Lane, Cold Spring Harbor Laboratory (1988), Cold Spring Harbor, N.Y. One skilled in the art will also appreciate that binding fragments or Fab fragments which mimic antibodies can also be prepared from genetic information by various procedures (Antibody Engineering: A Practical Approach (Borrebaeck, C., ed.), 1995, Oxford University Press, Oxford; J. Immunol. 149, 3914-3920 (1992)).

In one embodiment of the invention, antibodies to a specific domain of NAALADL2 are produced. In a specific embodiment, hydrophilic fragments of NAALADL2 are used as immunogens for antibody production.

In the production of antibodies, screening for the desired antibody can be accomplished by techniques known in the art, e.g. ELISA (enzyme-linked immunosorbent assay). For example, to select antibodies which recognize a specific domain of NAALADL2, one may assay generated hybridomas for a product which binds to a NAALADL2 fragment containing such domain. For selection of an antibody that specifically binds a first NAALADL2 homolog but which does not specifically bind to (or binds less avidly to) a second NAALADL2 homolog, one can select on the basis of positive binding to the first NAALADL2 homolog and a lack of binding to (or reduced binding to) the second NAALADL2 homolog. Similarly, for selection of an antibody that specifically binds NAALADL2 but which does not specifically bind to (or binds less avidly to) a different isoform of the same protein (such as a different glycoform having the same core peptide as NAALADL2), one can select on the basis of positive binding to NAALADL2 and a lack of binding to (or reduced binding to) the different isoform (e.g. a different glycoform). Thus, the present invention provides an antibody (such as a monoclonal antibody) that binds with greater affinity (for example at least 2-fold, such as at least 5-fold, particularly at least 10-fold greater affinity) to NAALADL2 than to a different isoform or isoforms (e.g. glycoforms) of NAALADL2.

Polyclonal antibodies which may be used in the methods of the invention are heterogeneous populations of antibody molecules derived from the sera of immunized animals. Unfractionated immune serum can also be used. Various procedures known in the art may be used for the production of polyclonal antibodies to NAALADL2, a fragment of NAALADL2, a NAALADL2-related polypeptide, or a fragment of a NAALADL2-related polypeptide. For example, one way is to purify polypeptides of interest or to synthesize the polypeptides of interest using, e.g. solid phase peptide synthesis methods well known in the art. See, e.g. Guide to Protein Purification, Murray P. Deutcher, ed., Meth. Enzymol. Vol 182 (1990); Solid Phase Peptide Synthesis, Greg B. Fields ed., Meth. Enzymol. Vol 289 (1997); Kiso et al., Chem. Pharm. Bull. (Tokyo) 38: 1192-99, 1990; Mostafavi et al., Biomed. Pept. Proteins Nucleic Acids 1: 255-60, 1995; Fujiwara et al., Chem. Pharm. Bull. (Tokyo) 44: 1326-31, 1996. The selected polypeptides may then be used to immunize by injection various host animals, including but not limited to rabbits, mice, rats, etc., to generate polyclonal or monoclonal antibodies. If NAALADL2 is purified by gel electrophoresis, NAALADL2 can be used for immunization with or without prior extraction from the polyacrylamide gel. Various adjuvants (i.e. immunostimulants) may be used to enhance the immunological response, depending on the host species, including, but not limited to, complete or incomplete Freund's adjuvant, a mineral gel such as aluminum hydroxide, surface active substance such as lysolecithin, pluronic polyol, a polyanion, a peptide, an oil emulsion, keyhole limpet hemocyanin, dinitrophenol, and an adjuvant such as BCG (bacille Calmette-Guerin) or corynebacterium parvum. Additional adjuvants are also well known in the art.

For preparation of monoclonal antibodies (mAbs) directed toward NAALADL2, a fragment of NAALADL2, a NAALADL2-related polypeptide, or a fragment of a NAALADL2-related polypeptide, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in Monoclonal Antibodies and Cancer Therapy, Alan R. Liss, Inc., pp. 77-96). Such antibodies may be of any immunoglobulin class including IgG, IgM, IgE, IgA, IgD and any subclass thereof. The hybridoma producing the mAbs of the invention may be cultivated *in vitro* or *in vivo.* In an additional embodiment of the invention, monoclonal antibodies can be produced in germ-free animals utilizing known technology (PCT/US90/02545).

The monoclonal antibodies include but are not limited to human monoclonal antibodies and chimeric monoclonal antibodies (e.g. human-mouse chimeras). A chimeric antibody is a molecule in which different portions are derived from different animal species, such as those having a human immunoglobulin constant region and a variable region derived from a murine mAb, (see, e.g. Cabilly et al., U.S. Patent No. 4,816,567; and Boss et al., U.S. Patent No. 4,816,397.) Humanized antibodies are antibody molecules from non-human species having one or more complementarity determining regions (CDRs) from the non-human species and a framework region from a human immunoglobulin molecule, (see, e.g. Queen, U.S. Patent No. 5,585,089.)

Chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al., 1988, Science 240:1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Canc. Res. 47:999-1005; Wood et al., 1985, Nature 314:446-449; and Shaw et al., 1988, J. Natl. Cancer Inst. 80:1553-1559; Morrison, 1985, Science 229:1202-1207; Oi et al., 1986, BioTechniques 4:214; U.S. Patent 5,225,539; Jones et al., 1986, Nature 321:552-525; Verhoeyan et al. (1988) Science 239:1534; and Beidler et al., 1988, J. Immunol. 141:4053-4060.

Completely human antibodies are particularly desirable for therapeutic treatment of human subjects. Such antibodies can be produced using transgenic mice which are incapable of expressing endogenous immunoglobulin heavy and light chain genes, but which can express human heavy and light chain genes. The transgenic mice are immunized in the normal fashion with a selected antigen, e.g. all or a portion of NAALADL2. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, *see, e.g.* U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA) and Genpharm (San Jose, CA) can be engaged to provide human antibodies directed against a selected antigen using technology similar to that described above.

Completely human antibodies which recognize a selected epitope can be generated using a technique referred to as "guided selection". In this approach a selected non-human monoclonal antibody, e.g. a mouse antibody, is used to guide the selection of a completely human antibody recognizing the same epitope. (Jespers et al. (1994) BioTechnology 12:899-903).

The antibodies of the present invention can also be generated by the use of phage display technology to produce and screen libraries of polypeptides for binding to a selected target. See, e.g. Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Patent No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. See, e.g. U.S. Patent No. 6,057,098. In particular, such phage can be utilized to display antigen binding domains expressed from a repertoire or combinatorial antibody library (e.g. human or murine). Phage expressing an antigen binding domain that binds the antigen of interest can be selected or identified with antigen, e.g. using labeled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Phage display methods that can be used to make the antibodies of the present invention include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994); PCT Application No. PCT/GB91/01134; PCT Publications WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g. as described in detail below. For example, techniques to recombinantly produce Fab, Fab' and F(ab')₂ fragments can also be employed using methods known in the art such as those disclosed in PCT publication WO 92/22324; Mullinax et al., BioTechniques 12(6):864-869 (1992); and Sawai et al., AJRI 34:26-34 (1995); and Better et al., Science 240:1041-1043 (1988).

Examples of techniques which can be used to produce single-chain Fvs and antibodies include those described in U.S. Patents 4,946,778 and 5,258,498; Huston et al., Methods in Enzymology 203:46-88 (1991); Shu et al., PNAS 90:7995-7999 (1993); and Skerra et al., Science 240:1038-1040 (1988).

The invention provides for the use of bispecific antibodies, which can be made by methods known in the art. Traditional production of full length bispecific antibodies is based on the coexpression of two immunoglobulin heavy chain-light chain pairs, where the two chains have different specificities (Milstein et al., 1983, Nature 305:537-539). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, published 13 May 1993, and in Traunecker et al., 1991, EMBO J. 10:3655-3659.

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690 published March 3, 1994. For further details for generating bispecific antibodies see, for example, Suresh et al., Methods in Enzymology, 1986, 121:210.

The invention provides functionally active fragments, derivatives or analogs of the anti-NAALADL2 immunoglobulin molecules. Functionally active means that the fragment, derivative or analog is able to elicit anti-anti-idiotype antibodies (*i.e.,* tertiary antibodies) that recognize the same antigen that is recognized by the antibody from which the fragment, derivative or analog is derived. Specifically, in a preferred embodiment the antigenicity of the idiotype of the immunoglobulin molecule may be enhanced by deletion of framework and CDR sequences that are C-terminal to the CDR sequence that specifically recognizes the antigen. To determine which CDR sequences bind the antigen, synthetic peptides containing the CDR sequences can be used in binding assays with the antigen by any binding assay method known in the art.

The present invention provides antibody fragments such as, but not limited to, F(ab')₂ fragments and Fab fragments. Antibody fragments which recognize specific epitopes may be generated by known techniques. F(ab')₂ fragments consist of the variable region, the light chain constant region and the CH1 domain of the heavy chain and are generated by pepsin digestion of the antibody molecule. Fab fragments are generated by reducing the disulfide bridges of the F(ab')₂ fragments. The invention also provides heavy chain and light chain dimers of the antibodies of the invention, or any minimal fragment thereof such as Fvs or single chain antibodies (SCAs) (*e.g.* as described in U.S. Patent 4,946,778; Bird, 1988, Science 242:423-42; Huston et al., 1988, Proc. Natl. Acad. Sci. USA 85:5879-5883; and Ward et al., 1989, Nature 334:544-54), or any other molecule with the same specificity as the antibody of the invention. Single chain antibodies are formed by linking the heavy and light chain fragments of the Fv region via an amino acid bridge, resulting in a single chain polypeptide. Techniques for the assembly of functional Fv fragments in E. *coli* may be used (Skerra et al., 1988, Science 242:1038-1041).

In other embodiments, the invention provides fusion proteins of the immunoglobulins of the invention (or functionally active fragments thereof), for example in which the immunoglobulin is fused via a covalent bond (e.g. a peptide bond), at either the N-terminus or the C-terminus to an amino acid sequence of another protein (or portion thereof, preferably at least 10, 20 or 50 amino acid portion of the protein) that is not the immunoglobulin. Preferably the immunoglobulin, or fragment thereof, is covalently linked to the other protein at the N-terminus of the constant domain. As stated above, such fusion proteins may facilitate purification, increase half-life *in vivo,* and enhance the delivery of an antigen across an epithelial barrier to the immune system.

The immunoglobulins of the invention include analogs and derivatives that are modified, i.e., by the covalent attachment of any type of molecule as long as such covalent attachment does not impair immunospecific binding. For example, but not by way of limitation, the derivatives and analogs of the immunoglobulins include those that have been further modified, e.g. by glycosylation, acetylation, pegylation, phosphylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, etc. Additionally, the analog or derivative may contain one or more non-classical amino acids.

The foregoing antibodies can be used in methods known in the art relating to the localization and activity of NAALADL2, e.g. for imaging this protein, measuring levels thereof in appropriate physiological samples, in diagnostic methods, etc.

### Production of Domain Antibodies to NAALADL2

References to antibodies herein embrace references to Domain Antibodies. Domain Antibodies (dAbs) are the smallest functional binding units of antibodies, corresponding to the variable regions of either the heavy (VH) or light (VL) chains of human antibodies. Domain Antibodies have a molecular weight of approximately 13 kDa. Domantis has developed a series of large and highly functional libraries of fully human VH and VL dAbs (more than ten billion different sequences in each library), and uses these libraries to select dAbs that are specific to therapeutic targets. In contrast to many conventional antibodies, Domain Antibodies are well expressed in bacterial, yeast, and mammalian cell systems. Further details of domain antibodies and methods of production thereof may be obtained by reference to US Patent 6,291,158; 6,582,915; 6,593,081; 6,172,197; 6,696,245; US Serial No. 2004/0110941; European patent application No. 1433846 and European Patents 0368684 and 0616640; WO05/035572, WO04/101790, WO04/081026, WO04/058821, WO04/003019 and WO03/002609.

### Expression of Affinity Reagents

### Expression of Antibodies

The antibodies of the invention can be produced by any method known in the art for the synthesis of antibodies, in particular, by chemical synthesis or by recombinant expression, and are preferably produced by recombinant expression techniques.

Recombinant expression of antibodies, or fragments, derivatives or analogs thereof, requires construction of a nucleic acid that encodes the antibody. If the nucleotide sequence of the antibody is known, a nucleic acid encoding the antibody may be assembled from chemically synthesized oligonucleotides (e.g. as described in Kutmeier et al., 1994, BioTechniques 17:242), which, briefly, involves the synthesis of overlapping oligonucleotides containing portions of the sequence encoding antibody, annealing and ligation of those oligonucleotides, and then amplification of the ligated oligonucleotides by PCR.

Alternatively, the nucleic acid encoding the antibody may be obtained by cloning the antibody. If a clone containing the nucleic acid encoding the particular antibody is not available, but the sequence of the antibody molecule is known, a nucleic acid encoding the antibody may be obtained from a suitable source (e.g. an antibody cDNA library, or cDNA library generated from any tissue or cells expressing the antibody) by PCR amplification using synthetic primers hybridizable to the 3' and 5' ends of the sequence or by cloning using an oligonucleotide probe specific for the particular gene sequence.

If an antibody molecule that specifically recognizes a particular antigen is not available (or a source for a cDNA library for cloning a nucleic acid encoding such an antibody), antibodies specific for a particular antigen may be generated by any method known in the art, for example, by immunizing an animal, such as a rabbit, to generate polyclonal antibodies or, for example, by generating monoclonal antibodies. Alternatively, a clone encoding at least the Fab portion of the antibody may be obtained by screening Fab expression libraries (e.g. as described in Huse et al., 1989, Science 246:1275-1281) for clones of Fab fragments that bind the specific antigen or by screening antibody libraries (see, e.g. Clackson et al., 1991, Nature 352:624; Hane et al., 1997 Proc. Natl. Acad. Sci. USA 94:4937).

Once a nucleic acid encoding at least the variable domain of the antibody molecule is obtained, it may be introduced into a vector containing the nucleotide sequence encoding the constant region of the antibody molecule (see, e.g. PCT Publication WO 86/05807; PCT Publication WO 89/01036; and U.S. Patent No. 5,122,464). Vectors containing the complete light or heavy chain for co-expression with the nucleic acid to allow the expression of a complete antibody molecule are also available. Then, the nucleic acid encoding the antibody can be used to introduce the nucleotide substitution(s) or deletion(s) necessary to substitute (or delete) the one or more variable region cysteine residues participating in an intrachain disulfide bond with an amino acid residue that does not contain a sulfhydyl group. Such modifications can be carried out by any method known in the art for the introduction of specific mutations or deletions in a nucleotide sequence, for example, but not limited to, chemical mutagenesis, in vitro site directed mutagenesis (Hutchinson et al., 1978, J. Biol. Chem. 253:6551), PCT based methods, etc.

In addition, techniques developed for the production of "chimeric antibodies" (Morrison et al., 1984, Proc. Natl. Acad. Sci. USA 81:851-855; Neuberger et al., 1984, Nature 312:604-608; Takeda et al., 1985, Nature 314:452-454) by splicing genes from a mouse antibody molecule of appropriate antigen specificity together with genes from a human antibody molecule of appropriate biological activity can be used. As described *supra,* a chimeric antibody is a molecule in which different portions are derived from different animal species, such as those having a variable region derived from a murine mAb and a human antibody constant region, e.g. humanized antibodies.

Once a nucleic acid encoding an antibody molecule of the invention has been obtained, the vector for the production of the antibody molecule may be produced by recombinant DNA technology using techniques well known in the art. Thus, methods for preparing NAALADL2 by expressing nucleic acid containing the antibody molecule sequences are described herein. Methods which are well known to those skilled in the art can be used to construct expression vectors containing an antibody molecule coding sequences and appropriate transcriptional and translational control signals. These methods include, for example, *in vitro* recombinant DNA techniques, synthetic techniques, and *in vivo* genetic recombination. See, for example, the techniques described in Sambrook et al. (1990, Molecular Cloning, A Laboratory Manual, 2nd Ed., Cold Spring Harbor Laboratory, Cold Spring Harbor, NY) and Ausubel et al. (eds., 1998, Current Protocols in Molecular Biology, John Wiley & Sons, NY).

The expression vector is transferred to a host cell by conventional techniques and the transfected cells are then cultured by conventional techniques to produce an antibody of the invention.

The host cells used to express a recombinant antibody of the invention may be either bacterial cells such as *Escherichia coli,* or, preferably, eukaryotic cells, especially for the expression of whole recombinant antibody molecule. In particular, mammalian cells such as Chinese hamster ovary cells (CHO), in conjunction with a vector such as the major intermediate early gene promoter element from human cytomegalovirus are an effective expression system for antibodies (Foecking et al., 1986, Gene 45:101; Cockett et al., 1990, BioTechnology 8:2).

A variety of host-expression vector systems may be utilized to express an antibody molecule of the invention. Such host-expression systems represent vehicles by which the coding sequences of interest may be produced and subsequently purified, but also represent cells which may, when transformed or transfected with the appropriate nucleotide coding sequences, express the antibody molecule of the invention *in situ.* These include but are not limited to microorganisms such as bacteria (e.g. E. *coli, B. subtilis*) transformed with recombinant bacteriophage DNA, plasmid DNA or cosmid DNA expression vectors containing antibody coding sequences; yeast (e.g. *Saccharomyces, Pichia*) transformed with recombinant yeast expression vectors containing antibody coding sequences; insect cell systems infected with recombinant virus expression vectors (e.g. baculovirus) containing the antibody coding sequences; plant cell systems infected with recombinant virus expression vectors (e.g. cauliflower mosaic virus, CaMV; tobacco mosaic virus, TMV) or transformed with recombinant plasmid expression vectors (e.g. Ti plasmid) containing antibody coding sequences; or mammalian cell systems (e.g. COS, CHO, BHK, 293, 3T3 cells) harboring recombinant expression constructs containing promoters derived from the genome of mammalian cells (e.g. metallothionein promoter) or from mammalian viruses (e.g. the adenovirus late promoter; the vaccinia virus 7.5K promoter).

In bacterial systems, a number of expression vectors may be advantageously selected depending upon the use intended for the antibody molecule being expressed. For example, when a large quantity of such a protein is to be produced, for the generation of pharmaceutical compositions comprising an antibody molecule, vectors which direct the expression of high levels of fusion protein products that are readily purified may be desirable. Such vectors include, but are not limited, to the E. *coli* expression vector pUR278 (Ruther et al., 1983, EMBO J. 2:1791), in which the antibody coding sequence may be ligated individually into the vector in frame with the *lac* Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. The pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter). In mammalian host cells, a number of viral-based expression systems (e.g. an adenovirus expression system) may be utilized.

As discussed above, a host cell strain may be chosen which modulates the expression of the inserted sequences, or modifies and processes the gene product in the specific fashion desired. Such modifications (e.g. glycosylation) and processing (e.g. cleavage) of protein products may be important for the function of the protein.

For long-term, high-yield production of recombinant antibodies, stable expression is preferred. For example, cell lines that stably express an antibody of interest can be produced by transfecting the cells with an expression vector comprising the nucleotide sequence of the antibody and the nucleotide sequence of a selectable (e.g. neomycin or hygromycin), and selecting for expression of the selectable marker. Such engineered cell lines may be particularly useful in screening and evaluation of compounds that interact directly or indirectly with the antibody molecule.

The expression levels of the antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain should be placed before the heavy chain to avoid an excess of toxic free heavy chain (Proudfoot, 1986, Nature 322:52; Kohler, 1980, Proc. Natl. Acad. Sci. USA 77:2197). The coding sequences for the heavy and light chains may comprise cDNA or genomic DNA.

Once the antibody molecule of the invention has been recombinantly expressed, it may be purified by any method known in the art for purification of an antibody molecule, for example, by chromatography (e.g. ion exchange chromatography, affinity chromatography such as with protein A or specific antigen, and sizing column chromatography), centrifugation, differential solubility, or by any other standard technique for the purification of proteins.

Alternatively, any fusion protein may be readily purified by utilizing an antibody specific for the fusion protein being expressed. For example, a system described by Janknecht et al. allows for the ready purification of non-denatured fusion proteins expressed in human cell lines (Janknecht et al., 1991, Proc. Natl. Acad. Sci. USA 88:8972-897). In this system, the gene of interest is subcloned into a vaccinia recombination plasmid such that the open reading frame of the gene is translationally fused to an amino-terminal tag consisting of six histidine residues. The tag serves as a matrix binding domain for the fusion protein. Extracts from cells infected with recombinant vaccinia virus are loaded onto Ni²⁺ nitriloacetic acid-agarose columns and histidine-tagged proteins are selectively eluted with imidazole-containing buffers.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) is present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (*e.g.* in sandwich assays) may interfere with one another sterically, etc., assay performance of an antibody may be a more important measure than absolute affinity and specificity of an antibody.

Those skilled in the art will recognize that many approaches can be taken in producing antibodies or binding fragments and screening and selecting for affinity and specificity for the various polypeptides, but these approaches do not change the scope of the invention.

For therapeutic applications, antibodies (particularly monoclonal antibodies) may suitably be human or humanized animal (e.g. mouse) antibodies. Animal antibodies may be raised in animals using the human protein (e.g. NAALADL2) as immunogen. Humanisation typically involves grafting CDRs identified thereby into human framework regions. Normally some subsequent retromutation to optimize the conformation of chains is required. Such processes are known to persons skilled in the art.

### Affinity Reagent Modifications

In one example, anti-NAALADL2 affinity reagents such as antibodies or fragments thereof are conjugated to a diagnostic moiety (such as a detectable label) or a therapeutic moiety. The antibodies can be used for diagnosis or to determine the efficacy of a given treatment regimen. Detection can be facilitated by coupling the antibody to a detectable substance (label). Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. Patent No. 4,741,900 for metal ions which can be conjugated to antibodies for use as diagnostics according to the present disclosure. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include ¹²⁵I, ¹³¹I, ¹¹¹In and ⁹⁹Tc. ⁶⁸Ga may also be employed.

As indicated above affinity reagents, such as antibodies, may be conjugated to a therapeutic moiety, such as a cytotoxin, a drug (e.g. an immunosuppressant) or a radiotoxin. Such conjugates are referred to herein as "immunoconjugates". Immunoconjugates that include one or more cytotoxins are referred to as "immunotoxins". A cytotoxin or cytotoxic agent includes any agent that is detrimental to (e.g. kills) cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents also include, for example, antimetabolites (e.g. methotrexate, 6-mercaptopurine, 6-thioguanine, cytarabine, 5-fluorouracil decarbazine), alkylating agents (e.g. mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g. daunorubicin (formerly daunomycin) and doxorubicin), antibiotics (e.g. dactinomycin (formerly actinomycin), bleomycin, mithramycin, and anthramycin (AMC)), and anti-mitotic agents (e.g. vincristine and vinblastine).

Other preferred examples of therapeutic cytotoxins that can be conjugated to an antibody include duocarmycins, calicheamicins, maytansines and auristatins, and derivatives thereof. An example of a calicheamicin antibody conjugate is commercially available (Mylotarg®; American Home Products).

Cytotoxins can be conjugated to antibodies using linker technology available in the art. Examples of linker types that have been used to conjugate a cytotoxin to an antibody include, but are not limited to, hydrazones, thioethers, esters, disulfides and peptide-containing linkers. A linker can be chosen that is, for example, susceptible to cleavage by low pH within the lysosomal compartment or susceptible to cleavage by proteases, such as proteases preferentially expressed in tumor tissue such as cathepsins (e.g. cathepsins B, C, D).

Examples of cytotoxins are described, for example, in U.S. Patent Nos. 6,989,452, 7,087,600, and 7,129,261, and in PCT Application Nos. PCT/US2002/17210, PCT/US2005/017804, PCT/US2006/37793, PCT/US2006/060050, PCT/US2006/060711, WO2006/110476, and in U.S. Patent Application No. 60/891,028. For further discussion of types of cytotoxins, linkers and methods for conjugating therapeutic agents to antibodies, see also Saito, G. et al. (2003) Adv. Drug Deliv. Rev. 55:199-215; Trail, P.A. et al. (2003) Cancer Immunol. Immunother. 52:328-337; Payne, G. (2003) Cancer Cell 3:207-212; Allen, T.M. (2002) Nat. Rev. Cancer 2:750-763; Pastan, I. and Kreitman, R. J. (2002) Curr. Opin. Investig. Drugs 3:1089-1091; Senter, P.D. and Springer, C.J. (2001) Adv. Drug Deliv. Rev. 53:247-264.

Affinity reagents can also be conjugated to a radioactive isotope to generate cytotoxic radiopharmaceuticals, also referred to as radioimmunoconjugates. Examples of radioactive isotopes that can be conjugated to antibodies for use diagnostically or therapeutically include, but are not limited to, iodine 131, indium 111, yttrium90 and lutetium 177. Methods for preparing radioimmunoconjugates are established in the art. Examples of radioimmunoconjugates are commercially available, including Zevalin® (IDEC Pharmaceuticals) and Bexxar® (Corixa Pharmaceuticals), and similar methods can be used to prepare radioimmunoconjugates using the antibodies.

Affinity reagents can also be conjugated to a phthalocyanine dye referred to hereafter as phthalocyanineconjugates. Examples of phthalocyanine dyes that can be conjugated to antibodies for use diagnostically or therapeutically include, but are not limited to, IR700. Methods for preparing phthalocyanineconjugates are described, for example, in Mitsunaga M, Ogawa M, Kosaka N, Rosenblum LT, Choyke PL and Kobayashi H (2011) Nat Med. 2011 Nov 6. doi: 10.1038/nm.2554.

The conjugates can be used to modify a given biological response, and the drug moiety is not to be construed as limited to classical chemical therapeutic agents. For example, the drug moiety may be a protein or polypeptide possessing a desired biological activity. Such proteins may include, for example, an enzymatically active toxin, or active fragment thereof, such as abrin, ricin A, pseudomonas exotoxin, or diphtheria toxin; a protein such as tumor necrosis factor or interferon-y; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors. Senter P.D. (2009) Curr. Opin. Chem. Biol. 13(3):235-244; Kovtun et a/. (2010) Cancer Res. 70(6):2528-2537.

Techniques for conjugating such therapeutic moieties to antibodies are well known, see, e.g. Arnon et al., "Monoclonal Antibodies For Immunotargeting Of Drugs In Cancer Therapy" in Monoclonal Antibodies And Cancer Therapy, Reisfeld et al. (eds.), pp. 243-56 (Alan R. Liss, Inc. 1985); Hellstrom et al., "Antibodies For Drug Delivery," in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review" in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy" in Monoclonal Antibodies For Cancer Detection And Therapy, Baldwin et al. (eds.), pp. 303-16 (Academic Press 1985), and Thorpe et al., Immunol. Rev., 62:119-58 (1982).

Alternatively, an antibody can be conjugated to a second antibody to form an antibody heteroconjugate as described by Segal in U.S. Patent No. 4,676,980.

An antibody with or without a therapeutic moiety conjugated to it can be used as a therapeutic that is administered alone or in combination with cytotoxic factor(s) and/or cytokine(s).

The invention also provides for fully human, or humanised antibodies that induce antibody-directed cell-mediated cytotoxicity (ADCC). A fully human antibody is one in which the protein sequences are encoded by naturally occurring human immunoglobulin sequences, either from isolated antibody-producing human B-lymphocytes, or from transgenic murine B-lymphocytes of mice in which the murine immunoglobulin coding chromosomal regions have been replaced by orthologous human sequences. Transgenic antibodies of the latter type include, but are not restricted to, HuMab (Medarex, Inc, CA) and XenoMouse (Abgenix Inc., CA). A humanised antibody is one in which the constant region of a non-human antibody molecule of appropriate antigen specificity, is replaced by the constant region of a human antibody, preferably of the IgG subtype, with appropriate effector functions (Morrison et al., 1984, Proc. Natl. Acad. Sci. 81:851-855; Neuberger et al., 1984, Nature 312:604-608; Takeda et al., 1985, Nature 314:452-454). Appropriate effector functions include ADCC, which is a natural process by which fully-human antibodies or humanized antibodies, when bound to targets on the surface of cancer cells, switch on the cell killing properties of lymphocytes that are part of the normal immune system. These active lymphocytes, called Natural Killer (NK) cells, use a cytotoxic process to destroy living cells to which the antibodies are bound. ADCC activity may be detected and quantified by measuring release of Europium (Eu³⁺) from Eu³⁺ labelled, living cells in the presence of an antigen-specific antibody and peripheral blood mononuclear cells extracted from an immunocompetent, living human subject. The ADCC process is described in detail in Janeway Jr. C.A. et al., Immunobiology, 5th ed., 2001, Garland Publishing, ISBN 0-8153-3642-X; Pier G.B. et al., Immunology, Infection, and Immunity, 2004, p246-5; Albanell J. et al., Advances in Experimental Medicine and Biology, 2003, 532:p2153-68 and Weng, W.-K. et al., Journal of Clinical Oncology, 2003, 21:p 3940-3947. Suitable methods for the detection and quantification of ADCC can be found in Blomberg et al., Journal of Immunological Methods. 1986, 86:p225-9; Blomberg et al., Journal of Immunological Methods. 1986, 21;92:p117-23 and Patel & Boyd, Journal of Immunological Methods. 1995, 184:p29-38.

ADCC typically involves activation of NK cells and is dependent on the recognition of antibody-coated cells by Fc receptors on the surface of the NK cell. The Fc receptors recognize the Fc (crystalline) portion of antibodies such as IgG, bound specifically to the surface of a target cell. The Fc receptor that triggers activation of the NK cell is called CD16 or FcyRIIIa. Once the FcyRIIIa receptor is bound to the IgG Fc, the NK cell releases cytokines such as IFN-γ, and cytotoxic granules containing perforin and granzymes that enter the target cell and promote cell death by triggering apoptosis.

The induction of antibody-dependent cellular cytotoxicity (ADCC) by an antibody can be enhanced by modifications that alter interactions between the antibody constant region (Fc) and various receptors that are present on the surface of cells of the immune system. Such modifications include the reduction or absence of alpha1,6-linked fucose moieties in the complex oligosaccharide chains that are normally added to the Fc of antibodies during natural or recombinant synthesis in mammalian cells. In a preferred embodiment, non-fucosylated anti-NAALADL2 affinity reagents such as antibodies or fragments thereof are produced for the purpose of enhancing their ability to induce the ADCC response.

Techniques for reducing or ablating alpha 1,6-linked fucose moieties in the oligosaccharide chains of the Fc are well established. In one example, the recombinant antibody is synthesized in a cell line that is impaired in its ability to add fucose in an alpha 1,6 linkage to the innermost N-acetylglucosamine of the N-linked biantennary complex-type Fc oligosaccharides. Such cell lines include, but are not limited to, the rat hybridoma YB2/0, which expresses a reduced level of the alpha 1,6-fucosyltransferase gene, FUT8. Preferably, the antibody is synthesized in a cell line that is incapable of adding alpha 1,6-linked fucosyl moieties to complex oligosaccharide chains, due to the deletion of both copies of the FUT8 gene. Such cell lines include, but are not limited to, FUT8-/- CHO/DG44 cell lines. Techniques for synthesizing partially fucosylated, or non-fucosylated antibodies and affinity reagents are described in Shinkawa et al., J. Biol. Chem. 278:3466-34735 (2003); Yamane-Ohnuki et al., Biotechnology and Bioengineering 87: 614-22 (2004) and in WO00/61739 A1, WO02/31140 A1 and WO03/085107 A1. In a second example, the fucosylation of a recombinant antibody is reduced or abolished by synthesis in a cell line that has been genetically engineered to overexpress a glycoprotein-modifying glycosyl transferase at a level that maximizes the production of complex N-linked oligosaccharides carrying bisecting N-acetylglucosamine. For example, the antibody is synthesized in a Chinese Hamster Ovary cell line expressing the enzyme N-acetyl glucosamine transferase III (GnT III). Cell lines stably transfected with suitable glycoprotein-modifying glycosyl transferases, and methods of synthesizing antibodies using these cells are described in WO99/54342.

A non-fucosylated antibody or affinity reagent can be used as a therapeutic that is administered alone or in combination with cytotoxic factor(s) and/or cytokine(s).

In a further modification, the amino acid sequences of the antibody Fc are altered in a way that enhances ADCC activation, without affecting ligand affinity. Examples of such modifications are described in Lazar et al., Proceedings of the National Academy of Sciences 2006, 103: p4005-4010; WO03/074679 and WO2007/039818. In these examples, substitution of amino acids in the antibody Fc, such as aspartate for serine at position 239, and isoleucine for glutamate at position 332, altered the binding affinity of an antibody for Fc receptors, leading to an increase in ADCC activation.

An antibody reagent with enhanced ADCC activation due to amino acid substitutions can be used as a therapeutic that is administered alone or in combination with cytotoxic factor(s) and/or cytokine(s).

The invention provides for bispecific molecules comprising at least one first binding specificity for a first target epitope (i.e. NAALADL2) and a second binding specificity for CD3. In other examples the second target epitope maybe present on the same target protein as that bound by the first binding specificity; or the second target epitope may be present of a different target protein to that bound by the first protein to that bound by the first binding specificity. The second target epitope may be present on the same cell as the first target epitope (i.e. NAALADL2); or the second target epitope may be present on a target which is not displayed by the cell which displays the first target epitope. As used herein, the term 'binding specificity' refers to a moiety comprising at least one antibody variable domain.

In one embodiment, the bispecific molecule is a BiTE (bispecific T-cell engager). In particular, the invention provides a bispecific antibody which comprises a first binding domain for NAALADL2 and a second binding domain for CD3.

These bispecific molecules target NAALADL2 expressing cells to CD3 expressing effector cells (e.g. CD3 expressing cytotoxic T cells) and trigger CD3-mediated effector cell activities, such as T cell clonal expansion and T cell cytotoxicity. The bispecific antibodies of the invention may have a total of either two or three antibody variable domains, wherein first portion of the bispecific antibody is capable of recruiting the activity of a human immune effector cell by specifically binding to an effector antigen located on the human immune effector cell, in which the effector antigen is the human CD3 antigen, said first portion consisting of one antibody variable domain, and a second portion of the bispecific antibody is capable of specifically binding to a target antigen other than the effector antigen e.g. NAALADL2, said target antigen being located on a target cell other than said human immune effector cell, and said second portion comprising one or two antibody variable domains.

The invention provides a bispecific antibody (preferably a BiTE) which binds to NAALADL2 and CD3 for the treatment of prostate cancer.

### Diagnosis of Cancer Including the disclosed diseases

According to the description , there is disclosed a method of detecting, diagnosing and/or screening for or monitoring the progression of cancer e.g. the disclosed diseases or of monitoring the effect of e.g. an anti-cancer drug or therapy directed towards the disclosed diseases in a subject which comprises detecting the presence or level of antibodies capable of immunospecific binding to NAALADL2, or one or more epitope-containing fragments thereof or which comprises detecting a change in the level thereof in said subject.

According to the description there is also disclosed a method of detecting, diagnosing and/or screening for cancer e.g. the disclosed diseases in a subject which comprises detecting the presence of antibodies capable of immunospecific binding to NAALADL2, or one or more epitope-containing fragments thereof in said subject, in which (a) the presence of an elevated level of antibodies capable of immunospecific binding to NAALADL2 or said one or more epitope-containing fragments thereof in said subject as compared with the level in a healthy subject or (b) the presence of a detectable level of antibodies capable of immunospecific binding to NAALADL2 or said one or more epitope-containing fragments thereof in said subject as compared with a corresponding undetectable level in a healthy subject indicates the presence of said cancer in said subject.

One particular method of detecting, diagnosing and/or screening for cancer, e.g. the disclosed diseases comprises:
bringing into contact with a biological sample to be tested NAALADL2, or one or more epitope-containing fragments thereof; and
detecting the presence of antibodies in the subject capable of immunospecific binding to NAALADL2, or one or more epitope-containing fragments thereof.

According to the description there is disclosed a method of monitoring the progression of cancer, e.g. the disclosed diseases or of monitoring the effect of e.g. an anti-cancer drug or therapy directed towards the disclosed diseases in a subject which comprises detecting the presence of antibodies capable of immunospecific binding to NAALADL2, or one or more epitope-containing fragments thereof in said subject at a first time point and at a later time point, the presence of an elevated or lowered level of antibodies capable of immunospecific binding to NAALADL2, or one or more epitope-containing fragments thereof in said subject at the later time point as compared with the level in said subject at said first time point, indicating the progression or regression of said cancer, or the effect or non-effect of said anti-cancer drug or therapy in said subject.

The presence of antibodies capable of immunospecific binding to NAALADL2, or one or more epitope-containing fragments thereof is typically detected by analysis of a biological sample obtained from said subject (exemplary biological samples are mentioned above, e.g. the sample is a sample of prostate, bladder, breast, esophagus, head and neck, colorectal, liver, lung, ovarian, gastric, uterus and pancreatic tissue, or else a sample of blood or saliva). The method typically includes the step of obtaining said biological sample for analysis from said subject. The antibodies that may be detected include IgA, IgM and IgG antibodies.

As described, test samples of e.g. prostate, bladder, breast, esophagus, head and neck, colorectal, liver, lung, ovarian, gastric, uterus or pancreatic tissue, serum, plasma or urine obtained from a subject suspected of having or known to have the disclosed diseases can be used for diagnosis or monitoring. In one example, a change in the abundance of NAALADL2 in a test sample relative to a control sample (from a subject or subjects free from the disclosed diseases) or a previously determined reference range indicates the presence of the disclosed diseases. In another example, the relative abundance of NAALADL2 in a test sample compared to a control sample or a previously determined reference range indicates a subtype of the disclosed diseases (e.g. prostate adenocarcinoma, transitional cell carcinoma, inflammatory breast cancer, familial or sporadic colorectal cancer, squamous cell head and neck carcinoma, hepatocellular carcinoma, squamous cell lung carcinoma, small cell carcinoma, endocrine tumours of the pancreas, ovarian adenocarcinoma, squamous cell esophagus carcinoma and gastric adenocarcinoma). In yet another example, the relative abundance of NAALADL2 in a test sample relative to a control sample or a previously determined reference range indicates the degree or severity of the disclosed diseases (e.g. the likelihood for metastasis). In any of the aforesaid methods, detection of NAALADL2 may optionally be combined with detection of one or more of additional biomarkers for the disclosed diseases. Any suitable method in the art can be employed to measure the level of NAALADL2, including but not limited to the Preferred Technologies described herein, kinase assays, immunoassays to detect and/or visualize the NAALADL2 (e.g. Western blot, immunoprecipitation followed by sodium dodecyl sulfate polyacrylamide gel electrophoresis, immunocytochemistry, etc.). In a further example, a change in the abundance of mRNA encoding NAALADL2 in a test sample relative to a control sample or a previously determined reference range indicates the presence of the disclosed diseases. Any suitable hybridization assay can be used to detect NAALADL2 expression by detecting and/or visualizing mRNA encoding the NAALADL2 (e.g. Northern assays, dot blots, *in situ* hybridization, etc.).

In another example, labelled antibodies (or other affinity reagents), derivatives and analogs thereof, which specifically bind to NAALADL2 can be used for diagnostic purposes to detect, diagnose, or monitor the disclosed diseases. Preferably, the disclosed diseases are detected in an animal, more preferably in a mammal and most preferably in a human.

### Screening Assays

The disclosure describes methods for identifying agents (e.g. candidate compounds or test compounds) that bind to NAALADL2 or have a stimulatory or inhibitory effect on the expression or activity of NAALADL2. The disclosure also describes methods of identifying agents, candidate compounds or test compounds that bind to a NAALADL2-related polypeptide or a NAALADL2 fusion protein or have a stimulatory or inhibitory effect on the expression or activity of a NAALADL2-related polypeptide or a NAALADL2 fusion protein. Examples of agents, candidate compounds or test compounds include, but are not limited to, nucleic acids (e.g. DNA and RNA), carbohydrates, lipids, proteins, peptides, peptidomimetics, small molecules and other drugs. Agents can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam, 1997, Anticancer Drug Des. 12:145; U.S. Patent No. 5,738,996; and U.S. Patent No. 5,807,683).

Examples of methods for the synthesis of molecular libraries can be found in the art, for example in: DeWitt et al., 1993, Proc. Natl. Acad. Sci. USA 90:6909; Erb et al., 1994, Proc. Natl. Acad. Sci. USA 91:11422; Zuckermann et al., 1994, J. Med. Chem. 37:2678; Cho et al., 1993, Science 261:1303; Carrell et al., 1994, Angew. Chem. Int. Ed. Engl. 33:2059; Carell et al., 1994, Angew. Chem. Int. Ed. Engl. 33:2061; and Gallop et al., 1994, J. Med. Chem. 37:1233.

Libraries of compounds may be presented, e.g. presented in solution (e.g. Houghten, 1992, BioTechniques 13:412-421), or on beads (Lam, 1991, Nature 354:82-84), chips (Fodor, 1993, Nature 364:555-556), bacteria (U.S. Patent No. 5,223,409), spores (Patent Nos. 5,571,698; 5,403,484; and 5,223,409), plasmids (Cull et al., 1992, Proc. Natl. Acad. Sci. USA 89:1865-1869) or phage (Scott and Smith, 1990, Science 249:386-390; Devlin, 1990, Science 249:404-406; Cwirla et al., 1990, Proc. Natl. Acad. Sci. USA 87:6378-6382; and Felici, 1991, J. Mol. Biol. 222:301-310),.

In one example, agents that interact with (i.e. bind to) NAALADL2, a NAALADL2 fragment (e.g. a functionally active fragment), a NAALADL2-related polypeptide, a fragment of a NAALADL2-related polypeptide, or a NAALADL2 fusion protein are identified in a cell-based assay system. In accordance with this example, cells expressing NAALADL2, a fragment of a NAALADL2, a NAALADL2-related polypeptide, a fragment of the NAALADL2-related polypeptide, or a NAALADL2 fusion protein are contacted with a candidate compound or a control compound and the ability of the candidate compound to interact with the NAALADL2 is determined. If desired, this assay may be used to screen a plurality (e.g. a library) of candidate compounds. The cell, for example, can be of prokaryotic origin (e.g. *E. coli*) or eukaryotic origin (e.g. yeast or mammalian). Further, the cells can express NAALADL2, a fragment of NAALADL2, a NAALADL2-related polypeptide, a fragment of the NAALADL2-related polypeptide, or a NAALADL2 fusion protein endogenously or be genetically engineered to express NAALADL2, a fragment of NAALADL2, a NAALADL2-related polypeptide, a fragment of the NAALADL2-related polypeptide, or a NAALADL2 fusion protein. In certain instances, NAALADL2, a fragment of NAALADL2, a NAALADL2-related polypeptide, a fragment of the NAALADL2-related polypeptide, or a NAALADL2 fusion protein or the candidate compound is labeled, for example with a radioactive label (such as ³²P, ³⁵S, and ¹²⁵I) or a fluorescent label (such as fluorescein isothiocyanate, rhodamine, phycoerythrin, phycocyanin, allophycocyanin, o-phthaldehyde or fluorescamine) to enable detection of an interaction between NAALADL2 and a candidate compound. The ability of the candidate compound to interact directly or indirectly with NAALADL2, a fragment of a NAALADL2, a NAALADL2-related polypeptide, a fragment of a NAALADL2-related polypeptide, or a NAALADL2 fusion protein can be determined by methods known to those of skill in the art. For example, the interaction between a candidate compound and NAALADL2, a NAALADL2-related polypeptide, a fragment of a NAALADL2-related polypeptide, or a NAALADL2 fusion protein can be determined by flow cytometry, a scintillation assay, immunoprecipitation or western blot analysis.

In another example, agents that interact with (i.e. bind to) NAALADL2, a NAALADL2 fragment (e.g. a functionally active fragment), a NAALADL2-related polypeptide, a fragment of a NAALADL2-related polypeptide, or a NAALADL2 fusion protein are identified in a cell-free assay system. In accordance with this example, native or recombinant NAALADL2 or a fragment thereof, or a native or recombinant NAALADL2-related polypeptide or fragment thereof, or a NAALADL2-fusion protein or fragment thereof, is contacted with a candidate compound or a control compound and the ability of the candidate compound to interact with NAALADL2 or NAALADL2-related polypeptide, or NAALADL2 fusion protein is determined. If desired, this assay may be used to screen a plurality (e.g. a library) of candidate compounds. Preferably, NAALADL2, a NAALADL2 fragment, a NAALADL2-related polypeptide, a fragment of a NAALADL2-related polypeptide, or a NAALADL2-fusion protein is first immobilized, by, for example, contacting NAALADL2, a NAALADL2 fragment, a NAALADL2-related polypeptide, a fragment of a NAALADL2-related polypeptide, or a NAALADL2 fusion protein with an immobilized antibody (or other affinity reagent) which specifically recognizes and binds it, or by contacting a purified preparation of NAALADL2, a NAALADL2 fragment, a NAALADL2-related polypeptide, fragment of a NAALADL2-related polypeptide, or a NAALADL2 fusion protein with a surface designed to bind proteins. NAALADL2, a NAALADL2 fragment, a NAALADL2-related polypeptide, a fragment of a NAALADL2-related polypeptide, or a NAALADL2 fusion protein may be partially or completely purified (e.g. partially or completely free of other polypeptides) or part of a cell lysate. Further, NAALADL2, a NAALADL2 fragment, a NAALADL2-related polypeptide, or a fragment of a NAALADL2-related polypeptide may be a fusion protein comprising NAALADL2 or a biologically active portion thereof, or NAALADL2-related polypeptide and a domain such as glutathionine-S-transferase. Alternatively, NAALADL2, a NAALADL2 fragment, a NAALADL2-related polypeptide, a fragment of a NAALADL2-related polypeptide or a NAALADL2 fusion protein can be biotinylated using techniques well known to those of skill in the art (e.g. biotinylation kit, Pierce Chemicals; Rockford, IL). The ability of the candidate compound to interact with NAALADL2, a NAALADL2 fragment, a NAALADL2-related polypeptide, a fragment of a NAALADL2-related polypeptide, or a NAALADL2 fusion protein can be determined by methods known to those of skill in the art.

In another example, a cell-based assay system is used to identify agents that bind to or modulate the activity of a protein, such as an enzyme, or a biologically active portion thereof, which is responsible for the production or degradation of NAALADL2 or is responsible for the post-translational modification of NAALADL2. In a primary screen, a plurality (e.g. a library) of compounds are contacted with cells that naturally or recombinantly express: (i) NAALADL2, an isoform of NAALADL2, a NAALADL2 homolog, a NAALADL2-related polypeptide, a NAALADL2 fusion protein, or a biologically active fragment of any of the foregoing; and (ii) a protein that is responsible for processing of NAALADL2, a NAALADL2 isoform, a NAALADL2 homolog, a NAALADL2-related polypeptide, a NAALADL2 fusion protein, or a fragment in order to identify compounds that modulate the production, degradation, or post-translational modification of NAALADL2, a NAALADL2 isoform, a NAALADL2 homolog, a NAALADL2-related polypeptide, a NAALADL2 fusion protein or fragment. If desired, compounds identified in the primary screen can then be assayed in a secondary screen against cells naturally or recombinantly expressing NAALADL2. The ability of the candidate compound to modulate the production, degradation or post-translational modification of NAALADL2, isoform, homolog, NAALADL2-related polypeptide, or NAALADL2 fusion protein can be determined by methods known to those of skill in the art, including without limitation, flow cytometry, a scintillation assay, immunoprecipitation and western blot analysis.

In another example, agents that competitively interact with (i.e. bind to) NAALADL2, a NAALADL2 fragment, a NAALADL2-related polypeptide, a fragment of a NAALADL2-related polypeptide, or a NAALADL2 fusion protein are identified in a competitive binding assay. In accordance with this example, cells expressing NAALADL2, a NAALADL2 fragment, a NAALADL2-related polypeptide, a fragment of a NAALADL2-related polypeptide, or a NAALADL2 fusion protein are contacted with a candidate compound and a compound known to interact with NAALADL2, a NAALADL2 fragment, a NAALADL2-related polypeptide, a fragment of a NAALADL2-related polypeptide or a NAALADL2 fusion protein; the ability of the candidate compound to preferentially interact with NAALADL2, a NAALADL2 fragment, a NAALADL2-related polypeptide, a fragment of a NAALADL2-related polypeptide, or a NAALADL2 fusion protein is then determined. Alternatively, agents that preferentially interact with (i.e. bind to) NAALADL2, a NAALADL2 fragment, a NAALADL2-related polypeptide or fragment of a NAALADL2-related polypeptide are identified in a cell-free assay system by contacting NAALADL2, a NAALADL2 fragment, a NAALADL2-related polypeptide, a fragment of a NAALADL2-related polypeptide, or a NAALADL2 fusion protein with a candidate compound and a compound known to interact with NAALADL2, a NAALADL2-related polypeptide or a NAALADL2 fusion protein. As stated above, the ability of the candidate compound to interact with NAALADL2, a NAALADL2 fragment, a NAALADL2-related polypeptide, a fragment of a NAALADL2-related polypeptide, or a NAALADL2 fusion protein can be determined by methods known to those of skill in the art. These assays, whether cell-based or cell-free, can be used to screen a plurality (e.g. a library) of candidate compounds.

In another example, agents that modulate (i.e. upregulate or downregulate) the expression or activity of NAALADL2 or a NAALADL2-related polypeptide are identified by contacting cells (e.g. cells of prokaryotic origin or eukaryotic origin) expressing NAALADL2 or a NAALADL2-related polypeptide with a candidate compound or a control compound (e.g. phosphate buffered saline (PBS)) and determining the expression of NAALADL2, NAALADL2-related polypeptide, or NAALADL2 fusion protein, mRNA encoding NAALADL2, or mRNA encoding the NAALADL2-related polypeptide. The level of expression of NAALADL2, NAALADL2-related polypeptide, mRNA encoding NAALADL2, or mRNA encoding the NAALADL2-related polypeptide in the presence of the candidate compound is compared to the level of expression of NAALADL2, NAALADL2-related polypeptide, mRNA encoding NAALADL2, or mRNA encoding the NAALADL2-related polypeptide in the absence of the candidate compound (e.g. in the presence of a control compound). The candidate compound can then be identified as a modulator of the expression of NAALADL2, or the NAALADL2-related polypeptide based on this comparison. For example, when expression of NAALADL2 or mRNA is significantly greater in the presence of the candidate compound than in its absence, the candidate compound is identified as a stimulator of expression of NAALADL2 or mRNA. Alternatively, when expression of NAALADL2 or mRNA is significantly less in the presence of the candidate compound than in its absence, the candidate compound is identified as an inhibitor of the expression of NAALADL2 or mRNA. The level of expression of NAALADL2 or the mRNA that encodes it can be determined by methods known to those of skill in the art. For example, mRNA expression can be assessed by Northern blot analysis or RT-PCR, and protein levels can be assessed by western blot analysis.

In another example, agents that modulate the activity of NAALADL2 or a NAALADL2-related polypeptide are identified by contacting a preparation containing NAALADL2 or NAALADL2-related polypeptide or cells (e.g. prokaryotic or eukaryotic cells) expressing NAALADL2 or NAALADL2-related polypeptide with a test compound or a control compound and determining the ability of the test compound to modulate (e.g. stimulate or inhibit) the activity of NAALADL2 or NAALADL2-related polypeptide. The activity of NAALADL2 or a NAALADL2-related polypeptide can be assessed by detecting induction of a cellular signal transduction pathway of NAALADL2 or NAALADL2-related polypeptide (e.g. intracellular Ca²⁺, diacylglycerol, IP3, etc.), detecting catalytic or enzymatic activity of the target on a suitable substrate, detecting the induction of a reporter gene (e.g. a regulatory element that is responsive to NAALADL2 or a NAALADL2-related polypeptide and is operably linked to a nucleic acid encoding a detectable marker, e.g. luciferase), or detecting a cellular response, for example, cellular differentiation, or cell proliferation. Based on the present description, techniques known to those of skill in the art can be used for measuring these activities (see, e.g. U.S. Patent No. 5,401,639). The candidate compound can then be identified as a modulator of the activity of NAALADL2 or a NAALADL2-related polypeptide by comparing the effects of the candidate compound to the control compound. Suitable control compounds include phosphate buffered saline (PBS) and normal saline (NS).

In another example, agents that modulate (i.e. upregulate or downregulate) the expression, activity or both the expression and activity of NAALADL2 or a NAALADL2-related polypeptide are identified in an animal model. Examples of suitable animals include, but are not limited to, mice, rats, rabbits, monkeys, guinea pigs, dogs and cats. Preferably, the animal used represent a model of the disclosed diseases (e.g. xenografts of prostate cancer cell lines such as LNCaP, PC-3, and DU-145, xenografts of bladder cancer cell lines such as UCRU-BL-12, UCRU-BL-13 and UCRU-BL-14, Russell et al. Cancer Res. 1986 Apr;46(4 Pt 2):2035-40, xenografts of breast cancer cell lines such as MCF-7 (Ozzello L, Sordat M., Eur J Cancer. 1980;16:553-559) and MCF10AT (Miller et al., J Natl Cancer Inst. 1993;85:1725-1732) in nude or SCID mice, xenografts of human colorectal cancer cell lines such as MDA-MB-345 in oestrogen-deprived SCID mice, (Eccles et al. 1994 Cell Biophysics 24/25, 279), xenografts of head and neck cancer cell lines such as FaDu and HNX-OE, xenografts of liver cancer cell lines such as MHCC97 in nude mice, Tian et al., Br J. Cancer 1999 Nov;81(5):814-21, xenografts of non small cell lung cancer cell lines such as A549 and H460, xenografts of small cell lung cancer cell lines such as NCI-H345, xenografts of pancreatic cancer cell lines such as MIA PaCa-2 in nude mice, Marincola et al., J Surg Res 1989 Dec;47(6):520-9, xenografts of gastric cancer cell lines such as NCI-N87 in nude mice and xenografts of ovarian cancer cell lines such as OVCAR3 in nude mice, Ulla K. NÃssander et al., CANCER RESEARCH 52, 646-653. February1. 1992). These can be utilized to test compounds that modulate NAALADL2 levels, since the pathology exhibited in these models is similar to that of e.g. the disclosed diseases. In accordance with this example, the test compound or a control compound is administered (e.g. orally, rectally or parenterally such as intraperitoneally or intravenously) to a suitable animal and the effect on the expression, activity or both expression and activity of NAALADL2 or NAALADL2-related polypeptide is determined. Changes in the expression of NAALADL2 or a NAALADL2-related polypeptide can be assessed by the methods outlined above.

In yet another example, NAALADL2 or a NAALADL2-related polypeptide is used as a "bait protein" in a two-hybrid assay or three hybrid assay to identify other proteins that bind to or interact with NAALADL2 or a NAALADL2-related polypeptide (see, e.g. U.S. Patent No. 5,283,317; Zervos et al. (1993) Cell 72:223-232; Madura et al. (1993) J. Biol. Chem. 268:12046-12054; Bartel et al. (1993) BioTechniques 14:920-924; Iwabuchi et al. (1993) Oncogene 8:1693-1696; and PCT Publication No. WO 94/10300). As those skilled in the art will appreciate, such binding proteins are also likely to be involved in the propagation of signals by NAALADL2 as, for example, upstream or downstream elements of a signaling pathway involving NAALADL2.

This description further discloses novel agents identified by the above-described screening assays and uses thereof for treatments as described herein. In addition, the description also discloses the use of an agent which interacts with, or modulates the activity of, NAALADL2 in the manufacture of a medicament for the treatment of the disclosed diseases.

### Therapeutic Use of NAALADL2

The disclosure describes the treatment or prevention of various diseases and disorders by administration of a therapeutic compound. Such compounds include but are not limited to: NAALADL2, NAALADL2 analogs, NAALADL2-related polypeptides and derivatives and variants (including fragments) thereof; antibodies (or other affinity reagents) to the foregoing; nucleic acids encoding NAALADL2, NAALADL2 analogs, NAALADL2-related polypeptides and fragments thereof; antisense nucleic acids to a gene encoding NAALADL2 or a NAALADL2-related polypeptide; and modulator (e.g. agonists and antagonists) of a gene encoding NAALADL2 or a NAALADL2-related polypeptide. An important feature of the disclosure is the identification of genes encoding NAALADL2 involved in cancers such as the disclosed diseases. The disclosed diseases, for example, can be treated (e.g. to ameliorate symptoms or to retard onset or progression) or prevented by administration of a therapeutic compound that reduces function or expression of NAALADL2 in the serum or tissue of subjects having the disclosed diseases.

In one embodiment, one or more antibodies (or other affinity reagents) each specifically binding to NAALADL2 are administered alone or in combination with one or more additional therapeutic compounds or treatments.

A biological product such as an antibody (or other affinity reagent) is allogeneic to the subject to which it is administered. In one example, a human NAALADL2 or a human NAALADL2-related polypeptide, a nucleotide sequence encoding a human NAALADL2 or a human NAALADL2-related polypeptide, or an antibody (or other affinity reagent) to a human NAALADL2 or a human NAALADL2-related polypeptide, is administered to a human subject for therapy (e.g. to ameliorate symptoms or to retard onset or progression) or prophylaxis.

Without being limited by theory, it is conceived that the therapeutic activity of antibodies (or other affinity reagents) which specifically bind to NAALADL2 may be achieved through the phenomenon of Antibody Dependent Cell-mediated Cytotoxicity (ADCC) (see e.g. Janeway Jr. C.A. et al., Immunobiology, 5th ed., 2001, Garland Publishing, ISBN 0-8153-3642-X; Pier G.B. et al., Immunology, Infection, and Immunity, 2004, p246-5; Albanell J. et al., Advances in Experimental Medicine and Biology, 2003, 532:p2153-68 and Weng, W-K. et al., Journal of Clinical Oncology, 2003, 21:p 3940-3947).

### Treatment And Prevention Of The disclosed diseases

The disclosed diseases, for example, are treated or prevented by administration to a subject suspected of having or known to have one or more of the disclosed diseases or to be at risk of developing one or more of the disclosed diseases of a compound that modulates (i.e. increases or decreases) the level or activity (i.e. function) of NAALADL2 that is differentially present in the serum or tissue of subjects having one or more of the disclosed diseases compared with serum or tissue of subjects free from the disclosed diseases. In one example, the disclosed diseases are treated or prevented by administering to a subject suspected of having or known to have one or more of the disclosed diseases or to be at risk of developing the disclosed diseases a compound that upregulates (i.e. decreases) the level or activity (i.e. function) of NAALADL2 that is increased in the serum or tissue of subjects having one or more of the disclosed diseases. Examples of such a compound include, but are not limited to, NAALADL2 antisense oligonucleotides, ribozymes, antibodies (or other affinity reagents) directed against NAALADL2, and compounds that inhibit the enzymatic activity of NAALADL2. Other useful compounds e.g. NAALADL2 antagonists and small molecule NAALADL2 antagonists, can be identified using *in vitro* assays.

Cancer, e.g. the disclosed diseases, may also be treated or prevented by administration to a subject suspected of having or known to have such cancer, or to be at risk of developing such cancer, of a compound that downregulates the level or activity (i.e. function) of NAALADL2 that are increased in the serum or tissue of subjects having such cancer. Examples of such a compound include but are not limited to: NAALADL2, NAALADL2 fragments and NAALADL2-related polypeptides; nucleic acids encoding NAALADL2, a NAALADL2 fragment and a NAALADL2-related polypeptide (e.g. for use in gene therapy); and, for those NAALADL2 or NAALADL2-related polypeptides with enzymatic activity, compounds or molecules known to modulate that enzymatic activity. Other compounds that can be used, e.g. NAALADL2 agonists, can be identified using in *in vitro* assays.

In another example, therapy or prophylaxis is tailored to the needs of an individual subject. Thus, in specific examples, compounds that promote the level or function of NAALADL2 are therapeutically or prophylactically administered to a subject suspected of having or known to have cancer e.g. the disclosed diseases, in whom the levels or functions of NAALADL2 are absent or are decreased relative to a control or normal reference range. In further examples, compounds that promote the level or function of NAALADL2 are therapeutically or prophylactically administered to a subject suspected of having or known to have cancer e.g. the disclosed diseases in whom the levels or functions of NAALADL2 are increased relative to a control or to a reference range. In further examples, compounds that decrease the level or function of NAALADL2 are therapeutically or prophylactically administered to a subject suspected of having or known to have cancer e.g. the disclosed diseases in whom the levels or functions of NAALADL2 are increased relative to a control or to a reference range. In further examples, compounds that decrease the level or function of NAALADL2 are therapeutically or prophylactically administered to a subject suspected of having or known to have cancer e.g. the disclosed diseases in whom the levels or functions of NAALADL2 are decreased relative to a control or to a reference range. The change in NAALADL2 function or level due to the administration of such compounds can be readily detected, e.g. by obtaining a sample (e.g. blood or urine) and assaying *in vitro* the levels or activities of NAALADL2, or the levels of mRNAs encoding NAALADL2, or any combination of the foregoing. Such assays can be performed before and after the administration of the compound as described herein.

The compounds described herein include but are not limited to any compound, e.g. a small organic molecule, protein, peptide, antibody (or other affinity reagent), nucleic acid, etc. that restores the NAALADL2 profile towards normal. The compounds described herein may be given in combination with any other chemotherapy drugs.

### Therapeutic and Prophylactic Compositions and their Use

The description discloses methods of treatment (and prophylaxis) comprising administering to a subject an effective amount of a compound described herein (e.g. NAALADL2 protein, an affinity reagent capable of specific binding to NAALADL2 or a fragment thereof. or a nucleic acid encoding NAALADL2). In a particular example, the compound is substantially purified (e.g. substantially free from substances that limit its effect or produce undesired side-effects).

Formulations and methods of administration that can be employed when the compound comprises a nucleic acid are described above; additional appropriate formulations and routes of administration are described below.

Various delivery systems are known and can be used to administer a compound described herein, e.g. encapsulation in liposomes, microparticles, microcapsules, recombinant cells capable of expressing the compound, receptor-mediated endocytosis (see, e.g. Wu and Wu, 1987, J. Biol. Chem. 262:4429-4432), construction of a nucleic acid as part of a retroviral or other vector, etc. Methods of introduction can be enteral or parenteral and include but are not limited to intradermal, intramuscular, intraperitoneal, intravenous, subcutaneous, intranasal, epidural, and oral routes. The compounds may be administered by any convenient route, for example by infusion or bolus injection, by absorption through epithelial or mucocutaneous linings (e.g. oral mucosa, rectal and intestinal mucosa, etc.) and may be administered together with other biologically active agents. Administration can be systemic or local. In addition, it may be desirable to introduce the pharmaceutical compositions of the description into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g. by use of an inhaler or nebulizer, and formulation with an aerosolizing agent.

In one example a nucleic acid employed in the described methods may be delivered to the dermis, for example employing particle mediated epidermal delivery.

In a specific embodiment, it may be desirable to administer the pharmaceutical compositions locally to the area in need of treatment; this may be achieved, for example, and not by way of limitation, by local infusion during surgery, topical application, e.g. by injection, by means of a catheter, or by means of an implant, said implant being of a porous, non-porous, or gelatinous material, including membranes, such as sialastic membranes, or fibers. In one example, administration can be by direct injection into e.g. prostate, bladder, breast, esophagus, head and neck, colorectal, liver, lung, ovarian, gastric, uterus and pancreatic tissue or at the site (or former site) of a malignant tumor or neoplastic or pre-neoplastic tissue.

In another example, the compound can be delivered in a vesicle, in particular a liposome (see Langer, 1990, Science 249:1527-1533; Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; see generally *ibid*.)

In yet another embodiment, the compound can be delivered in a controlled release system. In one embodiment, a pump may be used (see Langer, *supra;* Sefton, 1987, CRC Crit. Ref. Biomed. Eng. 14:201; Buchwald et al., 1980, Surgery 88:507; Saudek et al., 1989, N. Engl. J. Med. 321:574). In another embodiment, polymeric materials can be used (see Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, J., 1983, Macromol. Sci. Rev. Macromol. Chem. 23:61; see also Levy et al., 1985, Science 228:190; During et al., 1989, Ann. Neurol. 25:351; Howard et al., 1989, J. Neurosurg. 71:105). In yet another embodiment, a controlled release system can be placed in proximity of the therapeutic target, e.g. the disclosed diseases thus requiring only a fraction of the systemic dose (see, e.g. Goodson, in Medical Applications of Controlled Release, supra, vol. 2, pp. 115-138 (1984)). Other controlled release systems are discussed in the review by Langer (1990, Science 249:1527-1533).

In one example where the compound is a nucleic acid encoding a protein, the nucleic acid can be administered *in vivo* to promote expression of its encoded protein, by constructing it as part of an appropriate nucleic acid expression vector and administering it so that it becomes intracellular, e.g. by use of a retroviral vector (see U.S. Patent No. 4,980,286), or by direct injection, or by use of microparticle bombardment (e.g. a gene gun; Biolistic, Dupont), or coating with lipids or cell-surface receptors or transfecting agents, or by administering it in linkage to a homeobox-like peptide which is known to enter the nucleus (see e.g. Joliot et al., 1991, Proc. Natl. Acad. Sci. USA 88:1864-1868), etc. Alternatively, a nucleic acid can be introduced intracellularly and incorporated within host cell DNA for expression, by homologous recombination.

The disclosure also provides pharmaceutical compositions. Such compositions comprise a therapeutically effective amount of a compound, and a pharmaceutically acceptable carrier. In one example, the term "pharmaceutically acceptable" means suitable for approval by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the compound, for example in purified form, together with a suitable amount of carrier so as to provide the form for proper administration to the subject. The formulation should suit the mode of administration.

In one example, for example where one or more antibodies are employed, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compounds described herein can be formulated as neutral or salt forms. Pharmaceutically acceptable salts, where appropriate, include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc.

The amount of the compound which will be effective in the treatment of cancer, for example, the disclosed diseases can be determined by standard clinical techniques. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each subject's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from *in vitro* or animal model test systems.

Suppositories generally contain active ingredient in the range of 0.5% to 10% by weight; oral formulations preferably contain 10% to 95% active ingredient.

The disclosure provides a pharmaceutical pack or kit comprising one or more containers filled with one or more of the ingredients of the pharmaceutical compositions described herein. Optionally associated with such container(s) can be a notice in the form prescribed by a governmental agency regulating the manufacture, use or sale of pharmaceuticals or biological products, which notice reflects (a) approval by the agency of manufacture, use or sale for human administration, (b) directions for use, or both.

Thus in example the kit comprises antibodies described herein, for example the antibodies may be lyophilized for reconstitution before administration or use. Where the kit is for use in therapy/treatment such as cancer the antibody or antibodies may be reconstituted with an isotonic aqueous solution, which may optionally be provided with the kit. In one example the kit may comprise a polypeptide such as an immunogenic polypeptide described herein, which may for example be lyophilized. The latter kit may further comprise an adjuvant for reconstiting the immunogenic polypeptide.

The disclosure also extends to a composition as described herein for example a pharmaceutical composition and/or vaccine composition for use in inducing an immune response in a subject.

In yet a further exampleexample, the description provides a medicament comprising, separately or together:
(a) an affinity reagents which binds to NAALADL2, and
(b) an anti-cancer agent or other active agent,
for simultaneous, sequential or separate administration in the treatment of cancer, preferably in the treatment of one of the disclosed diseases.

### Determining Abundance of NAALADL2 by Imaging Technology

An advantage of determining abundance of NAALADL2 by imaging technology may be that such a method is non-invasive (save that reagents may need to be administered) and there is no need to extract a sample from the subject.

Suitable imaging technologies include positron emission tomography (PET) and single photon emission computed tomography (SPECT). Visualisation of NAALADL2 using such techniques requires incorporation or binding of a suitable label e.g. a radiotracer such as ¹⁸F, ¹¹C or ¹²³I (see e.g. NeuroRx - The Journal of the American Society for Experimental NeuroTherapeutics (2005) 2(2), 348-360 and idem pages 361-371 for further details of the techniques). Radiotracers or other labels may be incorporated into NAALADL2 by administration to the subject (e.g. by injection) of a suitably labelled specific ligand. Alternatively they may be incorporated into a binding affinity reagent (e.g. antibody) specific for NAALADL2 which may be administered to the subject (e.g. by injection). For discussion of use of Affibodies for imaging see e.g. Orlova A, Magnusson M, Eriksson TL, Nilsson M, Larsson B, Hoiden-Guthenberg I, Widstrom C, Carlsson J, Tolmachev V, Stahl S, Nilsson FY, Tumor imaging using a picomolar affinity HER2 binding Affibody molecule, Cancer Res. 2006 Apr 15;66(8):4339-48).

### Diagnosis And Treatment Of Cancer Including The disclosed diseases Using Immunohistochemistrv

Immunohistochemistry is an excellent detection technique and may therefore be very useful in the diagnosis and treatment of cancer, including the disclosed diseases. Immunohistochemistry may be used to detect, diagnose, or monitor cancers such as those mentioned above, through the localization of NAALADL2 antigens in tissue sections by the use of labeled antibodies (or other affinity reagents), derivatives and analogs thereof, which specifically bind to NAALADL2, as specific reagents through antigen-antibody interactions that are visualized by a marker such as fluorescent dye, enzyme, radioactive element or colloidal gold.

The advancement of monoclonal antibody technology has been of great significance in assuring the place of immunohistochemistry in the modern accurate microscopic diagnosis of human neoplasms. The identification of disseminated neoplastically transformed cells by immunohistochemistry allows for a clearer picture of cancer invasion and metastasis, as well as the evolution of the tumour cell associated immunophenotype towards increased malignancy. Future antineoplastic therapeutical approaches may include a variety of individualized immunotherapies, specific for the particular immunophenotypical pattern associated with each individual patient's neoplastic disease. For further discussion see e.g. Bodey B, The significance of immunohistochemistry in the diagnosis and therapy of neoplasms, Expert Opin Biol Ther. 2002 Apr; 2(4):371-93.

The invention is illustrated by the following non-limiting examples.

### EXAMPLE 1: IDENTIFICATION OF NAALADL2 EXPRESSED IN PROSTATE CANCER, BREAST CANCER, COLORECTAL CANCER, GASTRIC CANCER AND PANCREATIC CANCER TISSUE SAMPLES USING LIQUID CHROMATOGRAPHY-MASS SPECTROMETRY (LC/MS)

### EXAMPLE 2: IMMUNOHISTOCHEMISTRY USING ANTIBODY TO NAALADL2

Using the following Reference Protocol, immunohistochemistry was performed on FFPE tumor and normal tissues using a goat polyclonal antibody to NAALADL2 (R&D Systems, UK).

### 2.1 MATERIALS AND METHODS

### 2.1.1 Materials

EnVision plus kits (K4006 and K4010) were from DAKO, CA, USA.
EZ-De-Wax was from BioGenex, CA, USA.
Tissue sections and arrays were from Biomax, MD, USA.

### 2.1.2 Deparaffinisation and Rehydration

Slides were heated for 2 h at 60°C in 50ml Falcons in a water bath with no buffer. Each Falcon tube had one slide or two slides back-to back with a long gel loading tip between them to prevent slides from sticking. Slides were deparaffinised in EZ-DeWax for 5 min in a black slide rack, then rinsed with 1 ml of the same DeWax solution, followed by a distilled water wash. Slides were placed in a coplin jar filled with water; the water was changed twice.

### 2.1.3 Antigen Retrieval (Microwave)

Water was exchanged for antigen retrieval solution = 1 x citrate buffer, pH 6 (DAKO). Antigen was retrieved by the microwave method. The slides in the plastic coplin jar in antigen retrieval solution were placed into an 800W microwave which was then heated on full power until antigen retrieval solution was boiling. The antigen retrieval solution was then left to simmer on low power for a further 10 mins, after which the plastic coplin jar was removed from the microwave and left to cool to room temperature for another 20 min. The lid was opened and samples taken out to rest on the bench. The slides were washed 1x5min with PBS-3T (0.5 L PBS + 3 drops of Tween-20) and the slides were placed in PBS.

### 2.1.4 Antigen Retrieval (Pressure cooker)

Water was exchanged for antigen retrieval solution = 1 x citrate buffer, pH 6 (DAKO). Antigen was retrieved by the pressure cooker method. The slides in the plastic coplin jar in antigen retrieval solution were placed into a pressure cooker which was then heated up to position 6 (Russell Hobbs 13407 Hob Electric Two Boiling Ring, Model 13407), 15-20 min into the incubation, the temperature was reduced to position 3 and left at that (when the temperature inside the pressure cooker was 117°C) for another 20-25 min. Then the hob was switched off and the cooker was placed onto the cold hob and the pressure was released by carefully moving the handle into the position between "open" and "closed". The whole system was left to release the pressure and to cool down for another 20 min. The lid was opened and samples taken out to rest on the bench. The slides were washed 1 x 5 min with PBS-3T (0.5 L PBS + 3 drops of Tween-20) and the slides were placed in PBS.

### 2.1.4 Tissue staining

Endogenous peroxide blockade was performed using solution supplied with EnVision+ kits. The slide was taken out of the coplin jar and the PBS around tissues was wiped. Excess PBS on top of tissue was removed by tipping the slide on one side and soaking wipes in drop of PBS accumulating at the edge of the tissue section. Peroxide solution was dropped to cover the whole tissue. 1-4 drops was enough to cover even large sections. When all samples were covered with peroxide block, the time was set to 5 min. The slides were rinsed with water from wash bottle and then 1 x 5 min with PBS-3T, 1 x 5 min with PBS. They were then left in coplin jar in PBS. The primary antibody was diluted with an Antibody diluent reagent (DAKO) to 2.5µg/ml. Excess PBS was wiped from slides, excess PBS from tissue sections was removed as above. 50-200µl of diluted primary antibody was applied to each section and/or tissue microarray; taking care to cover the whole tissue. The slide was gently tapped to distribute the antibody evenly over the section or a pipette tip was used over the top of the section. The slide was incubated for 45 min in moist chamber at room temperature. The antibody was rinsed off with PBS from wash bottle and the slides were mounted in the Shandon Coverplate system. Air bubbles between the slide and plastic coverplate were prevented by placing the coverplate into the coplin jar filled with PBS and gently sliding the slide with tissue sections into the coverplate. The slide was pulled out of the coplin j ar at the same time holding it tightly together with the coverplate. The assembled slide was placed into the rack, letting PBS trapped in the funnel and between the slide and coverplate to run through. Slides were washed with 2 x 2ml (or 4 x 1ml) PBS-3T, 1 x 2ml PBS. The corresponding peroxidase polymer was applied 2 x 2 drops per slide and incubated for 35 min at room temperature. The slides were washed as above. The DAB substrate was made up in dilution buffer; 2ml containing 2 drops of substrate was enough for 10 slides. The DAB reagent was applied to the slides by applying a few drops at a time. All of the DAB was distributed between the slides. The slides were incubated for 10 min. The slides were washed 1 x 2ml with PBS-3T, 1 x 2ml (or 2 x 1ml) with PBS, waiting until all PBS had gone through the slide. Hematoxylin (DAKO) was applied; 1ml was enough for 10 slides and slides were incubated for 1 min at room temperature. Funnels were filled with 2ml of water and let to run through. When slides were clear of the excess of hematoxylin, the system was disassembled; tissue sections and/or arrays were washed with water from the wash bottle and placed into black slide rack. EZ-DeWax for 5 min; then 95% ethanol for 2-5 min. Slides were left to dry then mounted in mounting media and covered with coverslip.

### 2.2 RESULTS

Immunohistochemical analysis revealed specific staining of tumor cells in prostate cancer, bladder cancer, breast cancer, esophagus cancer, head and neck cancer, colorectal cancer, liver cancer, lung cancer, ovarian cancer, gastric cancer and uterus cancer. At high magnification it was evident that most of the cells were heavily stained in the plasma membrane. Thus antibodies directed to NAALADL2 may have utility as therapeutics and diagnostics in these cancers and other cancer types showing expression of NAALADL2.

### EXAMPLE 3: T cell activation and specific lysis of NAALADL2 expressing cells

### Background:

In order to assess the possibility of a target being amenable to a BiTE approach (bispecific antibody fragment combining anti-CD3 binding epitope combined with a binding site for a specific antigen on a target cell or tissue), an assay was developed to test T cell activation with anti-CD3 and a monoclonal antibody specific for a target antigen of interest.

### Methods:

For this assay NAALADL2 is expressed on LnCAP cells. The cells were counted, centrifuged at 800 xg, resuspended in assay media (dye free RPMI + 10% ultralow Ig FBS - Invitrogen catalog #16250078) at 0.12 million cells per ml. 6,000 cells (50 ul) was added to each appropriate well of a 96 well flat bottom tissue culture plate. The plate was previously coated overnight with goat anti-mouse kappa from Southern Biotech (catalog # 1050-01) at 3 ug/ml in PBS. The excess antibody solution was removed from the plates prior to adding the LnCAP cells. Human CD8+ T cells (frozen) were purchased from AllCells catalog number PB009-3F. The T cells were thawed and washed according to manufacturer's directions. Cells were resuspended at 1,500,000 cells per ml in assay media. The T cells were added to the LnCAP cells in the 96 well anti-kappa coated plate at 150,000 cells per well. The NAALADL2 antibody (R&D catalog # MAB4665) was added to the appropriate wells at 4.3 ug/ml or 1.4 ug/ml. Functional grade anti-CD3 clone OKT3 (eBioscience catalog number 16-0037-85) was added to the appropriate wells at 4.3 or 1.4 ug/ml. Control wells received no antibody. The plate was incubated for 24 hours in a 5% CO₂, humidified tissue culture incubator at 37 degrees.

50 ul of supernatant from each well was transferred to a 96 well flat bottom plate. The media was analyzed for LDH according to the manufacturer's directions on the CytoTox 96® NonRadioactive Cytotoxicity (Cat #G1780) and read at OD 490 nm on a visible plate reader. The ODs for the replicate were averaged and used to calculate the % lysis. Maximum lysis was determined using provided detergent and lysing wells plated with 6000 LnCAP cells at the same time the assay was plated. The maximum lysis supernatant OD was 2.1. Spontaneous lysis was determined using the no antibody wells (T cells + LnCAP cells with no added anti-CD3 or anti-NAALADL2 monoclonal antibody).

### Results:

Figure 1 shows the specific lysis of LnCAP cells by anti-NAALADL2 monoclonal antibodies and that cell death was proportional to antibody concentration. Thus Anti-NAALADL2 monoclonal antibodies are able to induce T-cell cytotoxicity via activation by CD3.

### SEQUENCES

| **SEQ ID No** | **Description** | **Sequence** |
|---|---|---|
| 1 | Inactive N-acetylated-alpha-linked acidic dipeptidase-like protein 2 (NAALADL2) | |
| 2 | Isoform 2 of Inactive N-acetylated-alpha-linked acidic dipeptidase-like protein 2 (NAALADL2) | |
| 3 | N-acetylated alpha-linked acidic dipeptidase-like 2 (NAALADL2) (NM_207015) | |
| | | |
| 4 | Isoform 2 of Inactive N-acetylated-alpha-linked acidic dipeptidase-like protein 2 (NAALADL2)(AJ60739 5) | |
| 5 | NAALADL2 Peptide 1 | AEVIDVSYGMADDLK |
| 6 | NAALADL2 Peptide 2 | AEVIDVSYGMADDLKR |
| 7 | NAALADL2 Peptide 3 | AGFGGVLLYIDPCDLPK |
| 8 | NAALADL2 Peptide 4 | IEEMDPSFNLHETITK |
| 9 | NAALADL2 Peptide 5 | LQEESDYITHYTR |
| 10 | NAALADL2 Peptide 6 | MLNDILQDMEK |
| 11 | NAALADL2 Peptide 7 | NEACSSLELPNNEIR |
| 12 | NAALADL2 Peptide 8 | NILYHLDEK |
| 13 | NAALADL2 Peptide 9 | NLVQLYK |
| 14 | NAALADL2 Peptide 10 | SNLTSLLVQPISAPLVAK |
| 15 | NAALADL2 Peptide 11 | TIQAEDIK |
| 16 | NAALADL2 Peptide 12 | TKNEACSSLELPNNEIR |
| 17 | NAALADL2 Peptide 13 | TLEGPSFLSEAR |
| 18 | NAALADL2 Peptide 14 | VVSMQVQTVTK |
| 19 | NAALADL2 ECD (aa 143-795 of SEQ ID NO: 1) | |

## Claims

1. A bispecific antibody which binds to NAALADL2 and CD3 for use in the treatment of prostate cancer wherein NAALADL2 is expressed in said prostate cancer.

2. The bispecific antibody for use according to claim 1, wherein the bispecific antibody is a monoclonal antibody.

3. The bispecific antibody for use according to claim 1 or claim 2 wherein the bispecific antibody elicits antibody-dependent cellular cytotoxicity (ADCC) or complement dependent cytotoxicity (CDC) or T-cell cytotoxicity or induces apoptosis of cancer cells, kills or reduces the number of cancer stem cells and/or kills or reduces the number of circulating cancer cells.

## Patentansprüche

1. Bispezifischer Antikörper, der an NAALADL2 und CD3 bindet, für die Verwendung bei der Behandlung von Prostatakrebs, wobei NAALADL2 in dem Prostatakrebs exprimiert wird.

2. Bispezifischer Antikörper für die Verwendung nach Anspruch 1, wobei der bispezifische Antikörper ein monoklonaler Antikörper ist.

3. Bispezifischer Antikörper für die Verwendung nach Anspruch 1 oder Anspruch 2, wobei der bispezifische Antikörper antikörperabhängige zelluläre Zytotoxizität (ADCC) oder komplementabhängige Zytotoxizität (CDC) oder T-Zell-Zytotoxizität auslöst oder Apoptose von Krebszellen induziert, die Anzahl von Krebsstammzellen abtötet oder reduziert und/oder die Anzahl von zirkulierenden Krebszellen abtötet oder reduziert.

## Revendications

1. Anticorps bispécifique qui se lie à NAALADL2 et CD3 pour une utilisation dans le traitement du cancer de la prostate, NAALADL2 étant exprimée dans ledit cancer de la prostate.

2. Anticorps bispécifique pour une utilisation selon la revendication 1, ledit anticorps bispécifique étant un anticorps monoclonal.

3. Anticorps bispécifique pour une utilisation selon la revendication 1 ou la revendication 2, ledit anticorps bispécifique provoquant une cytotoxicité cellulaire dépendante de l'anticorps (ADCC) ou une cytotoxicité dépendante du complément (CDC) ou une cytotoxicité des lymphocytes T ou induisant l'apoptose des cellules cancéreuses, tuant ou réduisant le nombre de cellules souches cancéreuses et/ou tuant ou réduisant le nombre de cellules cancéreuses en circulation.


### SEQUENCE LISTING

<110> Oxford BioTherpeutics
<120> THERAPEUTIC AND DIAGNOSTIC TARGET
<130> OB00069
<160> 19
<170> PatentIn version 3.5
<210> 1
   <211> 795
   <212> PRT
   <213> Homo sapiens
<400> 1
   Met Gly Glu Asn Glu Ala Ser Leu Pro Asn Thr Ser Leu Gln Gly Lys
   1               5                   10                  15
   Lys Met Ala Tyr Gln Lys Val His Ala Asp Gln Arg Ala Pro Gly His
               20                  25                  30
   Ser Gln Tyr Leu Asp Asn Asp Asp Leu Gln Ala Thr Ala Leu Asp Leu
           35                  40                  45
   Glu Trp Asp Met Glu Lys Glu Leu Glu Glu Ser Gly Phe Asp Gln Phe
       50                  55                  60
   Gln Leu Asp Gly Ala Glu Asn Gln Asn Leu Gly His Ser Glu Thr Ile
   65                  70                  75                  80
   Asp Leu Asn Leu Asp Ser Ile Gln Pro Ala Thr Ser Pro Lys Gly Arg
                   85                  90                  95
   Phe Gln Arg Leu Gln Glu Glu Ser Asp Tyr Ile Thr His Tyr Thr Arg
               100                 105                 110
   Ser Ala Pro Lys Ser Asn Arg Cys Asn Phe Cys His Val Leu Lys Ile
           115                 120                 125
   Leu Cys Thr Ala Thr Ile Leu Phe Ile Phe Gly Ile Leu Ile Gly Tyr
       130                 135                 140
   Tyr Val His Thr Asn Cys Pro Ser Asp Ala Pro Ser Ser Gly Thr Val
   145                 150                 155                 160
   Asp Pro Gln Leu Tyr Gln Glu Ile Leu Lys Thr Ile Gln Ala Glu Asp
                   165                 170                 175
   Ile Lys Lys Ser Phe Arg Asn Leu Val Gln Leu Tyr Lys Asn Glu Asp
               180                 185                 190
   Asp Met Glu Ile Ser Lys Lys Ile Lys Thr Gln Trp Thr Ser Leu Gly
           195                 200                 205
   Leu Glu Asp Val Gln Phe Val Asn Tyr Ser Val Leu Leu Asp Leu Pro
       210                 215                 220
   Gly Pro Ser Pro Ser Thr Val Thr Leu Ser Ser Ser Gly Gln Cys Phe
   225                 230                 235                 240
   His Pro Asn Gly Gln Pro Cys Ser Glu Glu Ala Arg Lys Asp Ser Ser
                   245                 250                 255
   Gln Asp Leu Leu Tyr Ser Tyr Ala Ala Tyr Ser Ala Lys Gly Thr Leu
               260                 265                 270
   Lys Ala Glu Val Ile Asp Val Ser Tyr Gly Met Ala Asp Asp Leu Lys
           275                 280                 285
   Arg Ile Arg Lys Ile Lys Asn Val Thr Asn Gln Ile Ala Leu Leu Lys
       290                 295                 300
   Leu Gly Lys Leu Pro Leu Leu Tyr Lys Leu Ser Ser Leu Glu Lys Ala
   305                 310                 315                 320
   Gly Phe Gly Gly Val Leu Leu Tyr Ile Asp Pro Cys Asp Leu Pro Lys
                   325                 330                 335
   Thr Val Asn Pro Ser His Asp Thr Phe Met Val Ser Leu Asn Pro Gly
               340                 345                 350
   Gly Asp Pro Ser Thr Pro Gly Tyr Pro Ser Val Asp Glu Ser Phe Arg
           355                 360                 365
   Gln Ser Arg Ser Asn Leu Thr Ser Leu Leu Val Gln Pro Ile Ser Ala
       370                 375                 380
   Pro Leu Val Ala Lys Leu Ile Ser Ser Pro Lys Ala Arg Thr Lys Asn
   385                 390                 395                 400
   Glu Ala Cys Ser Ser Leu Glu Leu Pro Asn Asn Glu Ile Arg Val Val
                   405                 410                 415
   Ser Met Gln Val Gln Thr Val Thr Lys Leu Lys Thr Val Thr Asn Val
               420                 425                 430
   Val Gly Phe Val Met Gly Leu Thr Ser Pro Asp Arg Tyr Ile Ile Val
           435                 440                 445
   Gly Ser His His His Thr Ala His Ser Tyr Asn Gly Gln Glu Trp Ala
       450                 455                 460
   Ser Ser Thr Ala Ile Ile Thr Ala Phe Ile Arg Ala Leu Met Ser Lys
   465                 470                 475                 480
   Val Lys Arg Gly Trp Arg Pro Asp Arg Thr Ile Val Phe Cys Ser Trp
                   485                 490                 495
   Gly Gly Thr Ala Phe Gly Asn Ile Gly Ser Tyr Glu Trp Gly Glu Asp
               500                 505                 510
   Phe Lys Lys Val Leu Gln Lys Asn Val Val Ala Tyr Ile Ser Leu His
           515                 520                 525
   Ser Pro Ile Arg Gly Asn Ser Ser Leu Tyr Pro Val Ala Ser Pro Ser
       530                 535                 540
   Leu Gln Gln Leu Val Val Glu Lys Asn Asn Phe Asn Cys Thr Arg Arg
   545                 550                 555                 560
   Ala Gln Cys Pro Glu Thr Asn Ile Ser Ser Ile Gln Ile Gln Gly Asp
                   565                 570                 575
   Ala Asp Tyr Phe Ile Asn His Leu Gly Val Pro Ile Val Gln Phe Ala
               580                 585                 590
   Tyr Glu Asp Ile Lys Thr Leu Glu Gly Pro Ser Phe Leu Ser Glu Ala
           595                 600                 605
   Arg Phe Ser Thr Arg Ala Thr Lys Ile Glu Glu Met Asp Pro Ser Phe
       610                 615                 620
   Asn Leu His Glu Thr Ile Thr Lys Leu Ser Gly Glu Val Ile Leu Gln
   625                 630                 635                 640
   Ile Ala Asn Glu Pro Val Leu Pro Phe Asn Ala Leu Asp Ile Ala Leu
                   645                 650                 655
   Glu Val Gln Asn Asn Leu Lys Gly Asp Gln Pro Asn Thr His Gln Leu
               660                 665                 670
   Leu Ala Met Ala Leu Arg Leu Arg Glu Ser Ala Glu Leu Phe Gln Ser
           675                 680                 685
   Asp Glu Met Arg Pro Ala Asn Asp Pro Lys Glu Arg Ala Pro Ile Arg
       690                 695                 700
   Ile Arg Met Leu Asn Asp Ile Leu Gln Asp Met Glu Lys Ser Phe Leu
   705                 710                 715                 720
   Val Lys Gln Ala Pro Pro Gly Phe Tyr Arg Asn Ile Leu Tyr His Leu
                   725                 730                 735
   Asp Glu Lys Thr Ser Arg Phe Ser Ile Leu Ile Glu Ala Trp Glu His
               740                 745                 750
   Cys Lys Pro Leu Ala Ser Asn Glu Thr Leu Gln Glu Ala Leu Ser Glu
           755                 760                 765
   Val Leu Asn Ser Ile Asn Ser Ala Gln Val Tyr Phe Lys Ala Gly Leu
       770                 775                 780
   Asp Val Phe Lys Ser Val Leu Asp Gly Lys Asn
   785                 790                 795
<210> 2
   <211> 320
   <212> PRT
   <213> Homo sapiens
<400> 2
   Met Ala Tyr Gln Lys Val His Ala Asp Gln Arg Ala Pro Gly His Ser
   1               5                   10                  15
   Gln Tyr Leu Asp Asn Asp Asp Leu Gln Ala Thr Ala Leu Asp Leu Glu
               20                  25                  30
   Trp Asp Met Glu Lys Glu Leu Glu Glu Ser Gly Phe Asp Gln Phe Gln
           35                  40                  45
   Leu Asp Gly Ala Glu Asn Gln Asn Leu Gly His Ser Glu Thr Ile Asp
       50                  55                  60
   Leu Asn Leu Asp Ser Ile Gln Pro Ala Thr Ser Pro Lys Gly Arg Phe
   65                  70                  75                  80
   Gln Arg Leu Gln Glu Glu Ser Asp Tyr Ile Thr His Tyr Thr Arg Ser
                   85                  90                  95
   Ala Pro Lys Ser Asn Arg Cys Asn Phe Cys His Val Leu Lys Ile Leu
               100                 105                 110
   Cys Thr Ala Thr Ile Leu Phe Ile Phe Gly Ile Leu Ile Gly Tyr Tyr
           115                 120                 125
   Val His Thr Asn Cys Pro Ser Asp Ala Pro Ser Ser Gly Thr Val Asp
       130                 135                 140
   Pro Gln Leu Tyr Gln Glu Ile Leu Lys Thr Ile Gln Ala Glu Asp Ile
   145                 150                 155                 160
   Lys Lys Ser Phe Arg Asn Leu Val Gln Leu Tyr Lys Asn Glu Asp Asp
                   165                 170                 175
   Met Glu Ile Ser Lys Lys Ile Lys Thr Gln Trp Thr Ser Leu Gly Leu
               180                 185                 190
   Glu Asp Val Gln Phe Val Asn Tyr Ser Val Leu Leu Asp Leu Pro Gly
           195                 200                 205
   Pro Ser Pro Ser Thr Val Thr Leu Ser Ser Ser Gly Gln Cys Phe His
       210                 215                 220
   Pro Asn Gly Gln Pro Cys Ser Glu Glu Ala Arg Lys Asp Ser Ser Gln
   225                 230                 235                 240
   Asp Leu Leu Tyr Ser Tyr Ala Ala Tyr Ser Ala Lys Gly Thr Leu Lys
                   245                 250                 255
   Ala Glu Val Ile Asp Val Ser Tyr Gly Met Ala Asp Asp Leu Lys Arg
               260                 265                 270
   Ile Arg Lys Ile Lys Asn Val Thr Asn Gln Ile Ala Leu Leu Lys Leu
           275                 280                 285
   Gly Lys Leu Pro Leu Leu Tyr Lys Val Gly Pro Val Asn Val Ile Gln
       290                 295                 300
   Trp Phe Gly Gln Tyr Phe Ala Leu Phe Cys Trp Asn Tyr Met Leu Leu
   305                 310                 315                 320
<210> 3
   <211> 4912
   <212> DNA
   <213> Homo sapiens
<400> 3
   acagtagaaa gtcagaaggt cacaaagctt gcagggtaag tgacacaact tgaaactgct        60
   tggccctctt taaaaagaaa taataaaatg ggagagaatg aagcaagttt acctaacacg       120
   tctttgcaag gtaaaaagat ggcctatcag aaggtccatg cagatcaaag agctccagga 180
   cactcacagt acttagacaa tgatgacctt caagccactg cccttgactt agagtgggac 240
   atggagaagg aactagagga gtctggtttt gaccaattcc agctagacgg tgctgagaat 300
   cagaacctag ggcattcaga gactatagac ctcaatcttg attccattca accagcaact 360
   tcacccaaag gaaggttcca gagacttcaa gaagaatctg actacattac ccattataca 420
   cgatctgcac caaagagcaa tcgctgcaac ttttgccacg tcttaaaaat actttgcaca 480
   gccaccattt tatttatttt tgggattttg ataggttatt atgtacatac aaattgccct 540
   tcagatgctc catcttcagg aacagttgat cctcagttat atcaagagat tctcaagaca 600
   atccaggcag aagatattaa gaagtctttc agaaatttgg tacaactata taaaaatgaa 660
   gatgacatgg aaatttcaaa gaagattaag actcagtgga cctctttggg cctagaagat 720
   gtacagtttg taaattactc tgtgctgctt gatctgccag gcccttctcc cagcactgtg 780
   actctgagca gcagtggtca atgctttcat cctaatggcc agccttgcag tgaagaagcc 840
   agaaaagata gcagccaaga cctgctctat tcatatgcag cctattctgc caaaggaact 900
   ctcaaggctg aagtcatcga tgtgagttat ggaatggcag atgatttaaa aaggattagg 960
   aaaataaaaa acgtaacaaa tcagatcgca ctcctgaaat taggaaaatt gccactgctt 1020
   tataagcttt cctcattgga aaaggctgga tttggaggtg ttcttctgta tatcgatcct 1080
   tgtgatttgc caaagactgt gaatcctagc catgatacct tcatggtgtc actgaatcca 1140
   ggaggagacc cttctacgcc tggttaccca agtgtcgatg aaagttttag acaaagccga 1200
   tcaaacctca cctctctatt agtgcagccc atctctgcac ccctcgttgc aaaactgatc 1260
   tcttcgccaa aagctagaac caaaaatgaa gcgtgtagct ctctagagct tccaaataat 1320
   gaaataagag tcgtcagcat gcaagttcag acagtcacaa aattgaaaac agttactaat 1380
   gttgttggat ttgtaatggg cttgacatct ccagaccggt atatcatagt tggcagccat 1440
   catcacactg cacacagtta taatggacaa gaatgggcca gtagtactgc aataatcaca 1500
   gcgtttatcc gtgccttgat gtcaaaagtt aagagagggt ggagaccaga ccgaactatt 1560
   gttttctgtt cttggggagg aacagctttt ggcaatattg gctcatatga atggggagag 1620
   gatttcaaga aggttcttca gaaaaatgtt gtggcttata ttagcctcca cagtcccata 1680
   agggggaact ctagtctgta tcctgtagca tcaccatctc ttcagcaact ggtagtagag     1740
   aaaaataatt tcaactgtac cagaagagcc cagtgcccag aaaccaatat cagttctata 1800
   cagatacaag gtgatgctga ttatttcatc aaccatcttg gagttcccat cgtgcagttt 1860
   gcttacgagg acatcaaaac attagagggt ccaagttttc tctccgaggc ccgtttttct 1920
   acacgagcaa caaaaattga agaaatggat ccctctttca accttcatga aaccattact 1980
   aagctctcag gagaagtgat tttgcaaatt gccaacgaac ctgttctgcc ctttaatgca 2040
   cttgatatag ctttagaagt tcaaaacaac cttaaaggtg atcaacccaa cactcatcaa 2100
   ctgttagcca tggcgttacg cctgcgggag agtgctgaac tttttcagtc tgatgagatg 2160
   cgacctgcta atgatcccaa ggagagagca cccatccgca tccggatgct gaatgacatt 2220
   ctccaagaca tggagaaaag ctttctggta aagcaggcac caccaggttt ttatagaaac 2280
   atcctctacc accttgatga aaagacaagc cggttttcaa tacttataga ggcttgggaa 2340
   cactgcaaac cccttgcatc aaatgagacc cttcaagaag ccctgtcaga ggtgttgaac 2400
   agcattaatt cagctcaggt ttacttcaaa gcaggacttg atgtgttcaa gagtgtcttg 2460
   gatgggaaga attgagaaaa ctctgagcat ttttaaaagt ttgtttacaa ttccacaagc 2520
   aaaagctcta atttaaccag attttctgac attgaaggct tattttcccc aatggctttt 2580
   tgacaagtat aaagctatta ttacattgta ttttttaaat gtaaatatag aaagaacatt 2640
   ttgcacattt aatatttttc ttatatctac atttctgaat atgtaaagca agttattgaa 2700
   ataggactta agaattacct attaaaaaga aatctgatat ataaaaatat gtactatttt 2760
   aaggaaaaat ttccaagaag ttctggacta tctttgttcc tatggggagg gcatatagga 2820
   acacagttta ttctctctga tagagctatt aatgacttag tttctgtaaa agaaatggag 2880
   agttgatatg gttcagatta acttcactat taagtgttca atatgaagaa ttcaagtatt 2940
   ctgactgagt gattggttga cctaaaccac tttgaatgtt tctattttat gaaatgaagt 3000
   ttctcttctt aacacaacta gatgtagtaa tacactggtt atgaaattgt atttttttaa 3060
   gtattaatga aaaaagagcc ataagcattc caggagaaaa tctcaaggga gctacataga 3120
   gcaatttaaa tgcaaatttt tttcctaaca acttacaagg tgactagctt tgaaacccct 3180
   aatttgcctc agttgatttt ctaagaattt caggagtgat gtatgtctta agagggagaa 3240
   aaaaatattt cttattactt tttctcttgt ttctgttgga aacactgaag cagggactct 3300
   aaaatgaaag catctcacat tggttttctt tcttgtatct tttctgaaac tccttgctgt 3360
   aaggcagctt tctcagagta ctatatattt tcaacagtaa agtagcagag tttctctttg 3420
   aaacccaaaa tgtcttctaa agaatcaaat ttctatttct gcctctgaca aaaagaactt 3480
   actatgaaag aaatagttgt tttatcaata aaagcccctt aatattatga agaaactttt 3540
   catatttttt cttctttatc cttaattgtg actaagatgg aaatctagaa aatattcatg 3600
   cttctaagtt tctgcagtgt tactgtaaga gagtgtctcg tctacacttc agttcttctc 3660
   tcatgtggaa aactaagctt tttatattga cattgccatt gaatagctct agcaactatt 3720
   attattcccc caaaccctaa aattctacct attaggtgtc aggttaaagt tctaatttat 3780
   ctttagaaat ttatgtgaaa aatggttttc ctatactgga taaagcaatt ccccttagga 3840
   aacatagcct cagagtattt catataaatt ttctctatct aattcaaaca tatctcccca 3900
   tgatacaccg ccagagtgac attgcagaca gtctgtgtat gtttaaattt tcacttttta     3960
   tggatgagca taaacctagt cttagtttaa aggcaaacta aagttgaggg gaaataatta     4020
   atcaatactg attaattgat ttataaggtt caacactgtc taccctaaat aatttttata     4080
   tgctcaaggc aaagcaggga atagaggcaa ttagctagat aattcaagca gaaatccatt     4140
   ttcatcgaaa ttgcctgtgt gcacatattt ttagatgaaa aatgaaacta ccttacaata     4200
   ttattttttt aaccaatgta tttgtttgca aacatttgcc atgttgaaga gtgtgtatag     4260
   gaaaggtcta gaaataaata ggcttgtgca tacctaggca ttcatgctct ttcttcctct     4320
   tcagagatca tcagaaaaga ggtgggaata cacaagcagg tgcatgtaag cccaaaagct     4380
   agaaagcctt atatttaacc aaaggttgat gtgttcattc cagttattca cttacagatg     4440
   aaactaaaac aaataaaggc aagctattat cagtttggta gttcattatt ttagaacaaa     4500
   tttctaaatt aatttattcc cataacctac aatagcactg agaacagaaa acatgtttct     4560
   tcttcatttt gtttaaagat tgcgtaccta ggtaagtcac actgtataga taaaaacctt     4620
   cttctgtatt cctcacctct aaattatatg ttatttgcta tttgaaaact gtagttaact     4680
   atggtttttt attagtctat taaaagatac gtggagatat taaattatac ctaaagcaga     4740
   cgtccaacaa attctgtaca tgctgcttta catctttgca acaaacctct taactagcag     4800
   cattatttct atagtgtgat tacctgaggg tgcctccaaa tgtatatcat ttcactcccc     4860
   tcccctcccc acccccaata gattcaaata aataaaatcc tgagcaaatt aa             4912
<210> 4
   <211> 1580
   <212> DNA
   <213> Homo sapiens
<400> 4
   ggcccggcag ccgtaccgca gacgagcccg cgcccgcgcc gctcaggaaa tgagcattga 60
   ctattaggca cctggatgaa gactgggtcc agattgtcag tctcctgtaa cctgcagaag 120
   ccctccagtt gcactaggaa cattcaattg aagtaaaaag atggcctatc agaaggtcca 180
   tgcagatcaa agagctccag gacactcaca gtacttagac aatgatgacc ttcaagccac 240
   tgcccttgac ttagagtggg acatggagaa ggaactagag gagtctggtt ttgaccaatt 300
   ccagctagac ggtgctgaga atcagaacct agggcattca gagactatag acctcaatct 360
   tgattccatt caaccagcaa cttcacccaa aggaaggttc cagagacttc aagaagaatc 420
   tgactacatt acccattata cacgatctgc accaaagagc aatcgctgca acttttgcca 480
   cgtcttaaaa atactttgca cagccaccat tttatttatt tttgggattt tgataggtta 540
   ttatgtacat acaaattgcc cttcagatgc tccatcttca ggaacagttg atcctcagtt 600
   atatcaagag attctcaaga caatccaggc agaagatatt aagaagtctt tcagaaattt 660
   ggtacaacta tataaaaatg aagatgacat ggaaatttca aagaagatta agactcagtg 720
   gacctctttg ggcctagaag atgtacagtt tgtaaattac tctgtgctgc ttgatctgcc 780
   aggcccttct cccagcactg tgactctgag cagcagtggt caatgctttc atcctaatgg 840
   ccagccttgc agtgaagaag ccagaaaaga tagcagccaa gacctgctct attcatatgc 900
   agcctattct gccaaaggaa ctctcaaggc tgaagtcatc gatgtgagtt atggaatggc 960
   agatgattta aaaaggatta ggaaaataaa aaacgtaaca aatcagatcg cactcctgaa 1020
   attaggaaaa ttgccactgc tttataaggt tggtccagtg aatgttattc agtggtttgg 1080
   tcaatatttt gccttgtttt gttggaatta tatgcttttg tgagtgtgga gtgtgtgtgt 1140
   gcatataggt gtgtgagaga gagaagggga gaggaagaaa gagaggcaga gagtgtcaca 1200
   gaaagatggc ttttccacat tagaacattt taatttaaga tatttaagaa caatatattt 1260
   atgcccttat ttctttagag agaaaatacc ttaagtcagg taacactgag tttgtgggac 1320
   cttaataaaa ttggcatact cttcataatg gtacctatct ggaatagtaa aaaatgagaa 1380
   ccaccctgtg ttcatcttat gacatatgtg aaacttctaa tctattatca aatggactaa 1440
   ttatcatgtt ctctatgtta gacaagtatc tagatgattt acacccttta gtgattattt 1500
   tgtcaactat acaactacag ttactaactg tgatcaggat tttaattaaa atataattgc 1560
   taagagtaaa aaaaaaaaaa 1580
<210> 5
   <211> 15
   <212> PRT
   <213> Homo sapiens
<400> 5
   Ala Glu Val Ile Asp Val Ser Tyr Gly Met Ala Asp Asp Leu Lys
   1               5                   10                  15
<210> 6
   <211> 16
   <212> PRT
   <213> Homo sapiens
<400> 6
   Ala Glu Val Ile Asp Val Ser Tyr Gly Met Ala Asp Asp Leu Lys Arg
   1               5                   10                  15
<210> 7
   <211> 17
   <212> PRT
   <213> Homo sapiens
<400> 7
   Ala Gly Phe Gly Gly Val Leu Leu Tyr Ile Asp Pro Cys Asp Leu Pro
   1               5                   10                  15
   Lys
<210> 8
   <211> 16
   <212> PRT
   <213> Homo sapiens
<400> 8
   Ile Glu Glu Met Asp Pro Ser Phe Asn Leu His Glu Thr Ile Thr Lys
   1               5                   10                  15
<210> 9
   <211> 13
   <212> PRT
   <213> Homo sapiens
<400> 9
   Leu Gln Glu Glu Ser Asp Tyr Ile Thr His Tyr Thr Arg
   1               5                   10
<210> 10
   <211> 11
   <212> PRT
   <213> Homo sapiens
<400> 10
   Met Leu Asn Asp Ile Leu Gln Asp Met Glu Lys
   1               5                   10
<210> 11
   <211> 15
   <212> PRT
   <213> Homo sapiens
<400> 11
   Asn Glu Ala Cys Ser Ser Leu Glu Leu Pro Asn Asn Glu Ile Arg
   1 5 10 15
<210> 12
   <211> 9
   <212> PRT
   <213> Homo sapiens
<400> 12
   Asn Ile Leu Tyr His Leu Asp Glu Lys
   1 5
<210> 13
   <211> 7
   <212> PRT
   <213> Homo sapiens
<400> 13
   Asn Leu Val Gln Leu Tyr Lys
   1 5
<210> 14
   <211> 18
   <212> PRT
   <213> Homo sapiens
<400> 14
   Ser Asn Leu Thr Ser Leu Leu Val Gln Pro Ile Ser Ala Pro Leu Val
   1 5 10 15
   Ala Lys
<210> 15
   <211> 8
   <212> PRT
   <213> Homo sapiens
<400> 15
   Thr Ile Gln Ala Glu Asp Ile Lys
   1 5
<210> 16
   <211> 17
   <212> PRT
   <213> Homo sapiens
<400> 16
   Thr Lys Asn Glu Ala Cys Ser Ser Leu Glu Leu Pro Asn Asn Glu Ile
   1 5 10 15
   Arg
<210> 17
   <211> 12
   <212> PRT
   <213> Homo sapiens
<400> 17
   Thr Leu Glu Gly Pro Ser Phe Leu Ser Glu Ala Arg
   1 5 10
<210> 18
   <211> 11
   <212> PRT
   <213> Homo sapiens
<400> 18
   Val Val Ser Met Gln Val Gln Thr Val Thr Lys
   1 5 10
<210> 19
   <211> 653
   <212> PRT
   <213> Homo sapiens
<400> 19
   Gly Tyr Tyr Val His Thr Asn Cys Pro Ser Asp Ala Pro Ser Ser Gly
   1               5                   10                  15
   Thr Val Asp Pro Gln Leu Tyr Gln Glu Ile Leu Lys Thr Ile Gln Ala
               20                  25                  30
   Glu Asp Ile Lys Lys Ser Phe Arg Asn Leu Val Gln Leu Tyr Lys Asn
           35                  40                  45
   Glu Asp Asp Met Glu Ile Ser Lys Lys Ile Lys Thr Gln Trp Thr Ser
       50                  55                  60
   Leu Gly Leu Glu Asp Val Gln Phe Val Asn Tyr Ser Val Leu Leu Asp
   65                  70                  75                  80
   Leu Pro Gly Pro Ser Pro Ser Thr Val Thr Leu Ser Ser Ser Gly Gln
                   85                  90                  95
   Cys Phe His Pro Asn Gly Gln Pro Cys Ser Glu Glu Ala Arg Lys Asp
               100                 105                 110
   Ser Ser Gln Asp Leu Leu Tyr Ser Tyr Ala Ala Tyr Ser Ala Lys Gly
           115                 120                 125
   Thr Leu Lys Ala Glu Val Ile Asp Val Ser Tyr Gly Met Ala Asp Asp
       130                 135                 140
   Leu Lys Arg Ile Arg Lys Ile Lys Asn Val Thr Asn Gln Ile Ala Leu
   145                 150                 155                 160
   Leu Lys Leu Gly Lys Leu Pro Leu Leu Tyr Lys Leu Ser Ser Leu Glu
                   165                 170                 175
   Lys Ala Gly Phe Gly Gly Val Leu Leu Tyr Ile Asp Pro Cys Asp Leu
   180 185 190
   Pro Lys Thr Val Asn Pro Ser His Asp Thr Phe Met Val Ser Leu Asn
   195 200 205
   Pro Gly Gly Asp Pro Ser Thr Pro Gly Tyr Pro Ser Val Asp Glu Ser
   210 215 220
   Phe Arg Gln Ser Arg Ser Asn Leu Thr Ser Leu Leu Val Gln Pro Ile
   225 230 235 240
   Ser Ala Pro Leu Val Ala Lys Leu Ile Ser Ser Pro Lys Ala Arg Thr
   245 250 255
   Lys Asn Glu Ala Cys Ser Ser Leu Glu Leu Pro Asn Asn Glu Ile Arg
   260 265 270
   Val Val Ser Met Gln Val Gln Thr Val Thr Lys Leu Lys Thr Val Thr
   275 280 285
   Asn Val Val Gly Phe Val Met Gly Leu Thr Ser Pro Asp Arg Tyr Ile
   290 295 300
   Ile Val Gly Ser His His His Thr Ala His Ser Tyr Asn Gly Gln Glu
   305 310 315 320
   Trp Ala Ser Ser Thr Ala Ile Ile Thr Ala Phe Ile Arg Ala Leu Met
   325 330 335
   Ser Lys Val Lys Arg Gly Trp Arg Pro Asp Arg Thr Ile Val Phe Cys
   340 345 350
   Ser Trp Gly Gly Thr Ala Phe Gly Asn Ile Gly Ser Tyr Glu Trp Gly
   355 360 365
   Glu Asp Phe Lys Lys Val Leu Gln Lys Asn Val Val Ala Tyr Ile Ser
   370 375 380
   Leu His Ser Pro Ile Arg Gly Asn Ser Ser Leu Tyr Pro Val Ala Ser
   385 390 395 400
   Pro Ser Leu Gln Gln Leu Val Val Glu Lys Asn Asn Phe Asn Cys Thr
   405 410 415
   Arg Arg Ala Gln Cys Pro Glu Thr Asn Ile Ser Ser Ile Gln Ile Gln
   420 425 430
   Gly Asp Ala Asp Tyr Phe Ile Asn His Leu Gly Val Pro Ile Val Gln
   435 440 445
   Phe Ala Tyr Glu Asp Ile Lys Thr Leu Glu Gly Pro Ser Phe Leu Ser
   450 455 460
   Glu Ala Arg Phe Ser Thr Arg Ala Thr Lys Ile Glu Glu Met Asp Pro
   465 470 475 480
   Ser Phe Asn Leu His Glu Thr Ile Thr Lys Leu Ser Gly Glu Val Ile
   485 490 495
   Leu Gln Ile Ala Asn Glu Pro Val Leu Pro Phe Asn Ala Leu Asp Ile
   500 505 510
   Ala Leu Glu Val Gln Asn Asn Leu Lys Gly Asp Gln Pro Asn Thr His
   515 520 525
   Gln Leu Leu Ala Met Ala Leu Arg Leu Arg Glu Ser Ala Glu Leu Phe
   530 535 540
   Gln Ser Asp Glu Met Arg Pro Ala Asn Asp Pro Lys Glu Arg Ala Pro
   545 550 555 560
   Ile Arg Ile Arg Met Leu Asn Asp Ile Leu Gln Asp Met Glu Lys Ser
   565 570 575
   Phe Leu Val Lys Gln Ala Pro Pro Gly Phe Tyr Arg Asn Ile Leu Tyr
   580 585 590
   His Leu Asp Glu Lys Thr Ser Arg Phe Ser Ile Leu Ile Glu Ala Trp
   595 600 605
   Glu His Cys Lys Pro Leu Ala Ser Asn Glu Thr Leu Gln Glu Ala Leu
   610 615 620
   Ser Glu Val Leu Asn Ser Ile Asn Ser Ala Gln Val Tyr Phe Lys Ala
   625 630 635 640
   Gly Leu Asp Val Phe Lys Ser Val Leu Asp Gly Lys Asn
   645 650